# EUROPEAN PATENT APPLICATION

(11) **EP 4 032 974 A1**
(43) Date of publication of application: **27.07.2022**
(21) Application number: 20864564.8
(22) Date of filing: 21.09.2020
(51) Int. Cl.: C12N 5/0793, A61K 35/30, A61P 25/16, G01N 33/50

(54) **PRODUCTION METHOD FOR INDUCED DOPAMINERGIC NEURONAL PROGENITORS, USING DIRECT REPROGRAMMING**

(30) Priority: 20.09.2019 KR 20190116258
(71) Applicant: Korea Research Institute of Bioscience and Biotechnology, Daejeon 34141 (KR)
(72) Inventor: KIM, Janghwan, Daejeon 34141 (KR); LEE, Minhyung, Daejeon 34141 (KR); SON, Mi Young, Daejeon 34141 (KR); JEON, Young Joo, Daejeon 34141 (KR); BAEK, Areum, Daejeon 34141 (KR); LEE, Young Jeon, Daejeon 34141 (KR); SEO, Jincheol, Daejeon 34141 (KR); JUNG, Cho Rok, Daejeon 34141 (KR)
(74) Representative: Dehns
(86) International application number: PCT/KR2020/012721
(87) International publication number: WO 2021/054806

(57) **Abstract**

The present invention relates to a method for producing induced dopaminergic neuronal progenitors from adult cells using direct reprogramming, induced dopaminergic neuronal progenitors produced via the method and a use for same, wherein, as a result of having been directly reprogrammed from adult cells, the induced dopaminergic neuronal progenitors produced by means of the present invention can be transplanted inside a living body without the risk of oncogenicity, and have excellent proliferative capacity and dopaminergic neuronal differentiation potency, thus can be usefully utilized as a cell therapy product for Parkinson's disease.

## Description

### Technical Field

The present disclosure relates to a method for preparing induced dopaminergic neuronal progenitors through direct reprogramming from adult cells. In addition, the present disclosure relates to induced dopaminergic neuronal progenitors prepared through the method and uses thereof.

### Background Art

Parkinson's disease (PD) occurs due to the loss of dopaminergic neurons (DNs) in *substania nigra pars compacta* (SNpc), and it leads to a gradual deterioration of motor activity. Currently, the main process of treatment for PD patients is alleviation of the symptoms using drugs that increase dopamine concentration and electrical devices that directly stimulate neurons in the deep brain. Although this approach is effective in alleviating the motor symptoms of PD, it has a limitation in that the worsening of PD cannot be stopped.

Recently, as a novel PD treatment method, a cell replacement therapy was reported in which human fetal midbrain is used for transplantation of dopamine-secreting cells. In particular, this therapy improved motor symptoms even with discontinuation of drug administration in some patients, while not worsening non-motor symptoms for more than 18 years. In addition, in postmortem tissue analysis of transplant recipients, donor-derived DNs were shown to maintain a wide innervation in the striatum for up to 24 years after transplantation. These results demonstrated that long-term survival of donor-derived functional DNs in the brains of transplant recipients was associated with positive outcomes of PD treatment.

However, in a double-blind study performed in patients with severe symptomatic PD, temporary and mild clinical effects were merely observed. It was confirmed that the number of viable cells was small after termination of the administration of an immunosuppressant, which was due to the immune rejection of the transplanted tissue and is expected to be the main cause of failure of the clinical trial. Furthermore, graft-induced dyskinesia occurred in some recipients, and their brains included serotonin neurons in the graft. It also had ethical issues relating to the use of fetal tissues and technical issues of a limited amount of cells. Therefore, in order to improve the effectiveness of PD cell therapy regimen, a new method for obtaining homogeneous and low immunogenic DNs is needed.

Reprogramming technology is a technology that can convert the cell fate of somatic cells into induced pluripotent stem cells (iPSCs). Human iPSCs (hiPSCs) are a suitable source of cells for autologous cell therapy considering their ability to differentiate into all cell types and their unlimited self-renewal properties.

In pre-clinical experiments using pluripotent stem cell-derived dopaminergic neuron precursors (PSC-DPs) for the treatment of PD, the grafts showed good viability and effectively functioned as dopamine-secreting cells in rodent and non-human primate PD models. Despite such apparent efficacy, there is a possibility that undifferentiated PSCs may remain in differentiated cells, and due to the potential for tumorigenicity, there still remains the safety issue regarding PSC-derived dopaminergic neurons (PSC-DNs) in clinical use. In addition, it was reported that DPs differentiated from PSCs have limited proliferation ability and gradually disappear into DNs during repetitive subcultures. Therefore, for successful PD treatment, new cells that can proliferate more safely and stably are required.

Meanwhile, the direct reprogramming technology is another method for converting a cell fate, and may be performed by target cell specificity and/or ectopic expression of pluripotent factors. Interestingly, it was reported that direct reprogramming with at least one pluripotent factor can produce proliferable stem/progenitor cells. Importantly, it is becoming clear that cells reprogrammed by the pluripotent cell-specific factor-mediated direct reprogramming (PDR) method do not form tumors. In addition, human induced neural stem cells (hiNSCs) have the advantages that enable stable proliferation ability, long-term storage without changes in proliferation and differentiation during frozen storage, and ease of the differentiation process. Therefore, it is expected that PDR can overcome the limited proliferation and stability problems of PSC-DPs.

Previously, the present inventors had successfully produced mouse-induced dopaminergic neuronal precursors (miDPs) by a PDR method (Korean Patent Publication No. 10-2015-0015294). Thus, the present inventors performed experiments whether human adult cells are directly reprogrammed into human iDPs (hiDPs) by the introduction of pluripotent factors followed by activation of midbrain-specific signaling.

### Disclosure of Invention

### Technical Problem

An object of the present disclosure is to provide a method for preparing an induced dopaminergic neuron precursor, which enables successive subcultures from adult cells through direct reprogramming, has an excellent ability to be differentiated into dopaminergic neurons, and does not have the risk of tumorigenicity *in vivo.*

Another object of the present disclosure is to provide an induced dopaminergic neuronal progenitor prepared from the method described above.

Still another object of the present disclosure is to provide induced dopaminergic neuronal progenitors distinguishable from the pluripotent stem cell-derived dopaminergic neuronal progenitors.

Still another object of the present disclosure is to provide a pharmaceutical composition for preventing or treating Parkinson's disease.

Still another object of the present disclosure is to provide a method for preventing or treating Parkinson's disease.

Still another object of the present disclosure is to provide a use of the induced dopaminergic neuronal progenitors.

Still another object of the present disclosure is to provide a method for screening agents for preventing or treating Parkinson's disease.

Still another object of the present disclosure is to provide a mixture or medium composition for preparing induced dopaminergic neuronal progenitors.

Still another object of the present disclosure is to provide a method for preparing dopaminergic neurons.

### Solution to Problem

To achieve the above objects, the present disclosure provides a method for preparing induced dopaminergic neuronal progenitors (iDPs), which comprises a) introducing one or more genes selected from the group consisting of Oct4, Sox2, Klf4, and Myc into adult cells; b) culturing the cells in a medium containing EGF and FGF2; and c) culturing the cells in a medium containing FGF8, SHH, a Wnt signaling agonist, and a TGF-β inhibitor.

The present disclosure also provides dopaminergic neuronal progenitors prepared by the method described above.

The present disclosure also provides induced dopaminergic neuronal progenitors, in which (i) one or more genes selected from the group consisting of CORIN, FOXA2, and LMX1A exhibit reduced expression compared to dopaminergic neuronal progenitors derived from pluripotent stem cells; (ii) one or more genes selected from the group consisting of EN1, PAX2, PAX5, PAX8, and SPRY1 exhibit increased expression compared to dopaminergic neuronal progenitors derived from pluripotent stem cells; or (iii) the reduced expression of (i) and the increased expression of (ii) are exhibited.

The present disclosure also provides a pharmaceutical composition for preventing or treating Parkinson's disease containing the induced dopaminergic neuronal progenitors as an active ingredient.

The present disclosure also provides a method for preventing or treating Parkinson's disease, which comprises administering to a subject in a therapeutically effective amount the pharmaceutical composition.

The present disclosure also provides a use of the induced dopaminergic neuronal progenitors for preventing or treating Parkinson's disease.

The present disclosure also provides a use of the induced dopaminergic neuronal progenitors for the preparation of a medicament for preventing or treating Parkinson's disease.

The present disclosure also provides a method for screening agents for preventing or treating Parkinson's disease, which comprises treating the induced dopaminergic neuronal progenitors with a candidate material for preventing or treating Parkinson's disease.

The present disclosure also provides a composition for screening agents for preventing or treating Parkinson's disease, which contains the induced dopaminergic neuronal progenitors.

The present disclosure also provides a use of the induced dopaminergic neuronal progenitors for screening agents for preventing or treating Parkinson's disease.

The present disclosure also provides a mixture for preparing an induced dopaminergic neuronal progenitor, which contains human adult cells into which one or more genes selected from the group consisting of Oct4, Sox2, Klf4, and Myc are introduced; EGF; FGF2; a Wnt signaling agonist; and a TGF-β inhibitor.

The present disclosure also provides a mixture, which contains an induced dopaminergic neuronal progenitor, FGF8, SHH, a Wnt signaling agonist, and a TGF-β inhibitor.

The present disclosure also provides a medium composition for preparing an induced dopaminergic progenitor, which contains EGF, FGF2, a Wnt signaling agonist, and a TGF-β inhibitor.

The present disclosure also provides a medium composition for preparing or maintaining an induced dopaminergic progenitor, which contains FGF8, SHH, a Wnt signaling agonist, and a TGF-β inhibitor.

The present disclosure also provides a method for preparing a dopaminergic neuron, which comprises culturing the induced dopaminergic neuronal progenitor in a neuronal differentiation medium.

### Advantageous Effects of Invention

Due to the use of the patient's adult cells, the induced dopaminergic neuronal progenitors prepared by the present disclosure are little risk of side effects (e.g., immune rejection and dyskinesia) and there is no ethical problem. In addition, the induced dopaminergic neuronal progenitors of the present disclosure enable stable proliferation through successive subcultures, enable transplantation *in vivo* via direct reprogramming without the risk of tumorigenicity, and have an excellent differentiation ability into neurons, and are thus useful for cell therapy.

### Brief Description of Drawings

FIG. 1 shows a schematic diagram illustrating a combination of factors for developing a direct reprogramming protocol for hiDPs.
FIG. 2 shows representative bright field images of the results of direct reprogramming of hiDPs under each condition shown in FIG. 1. The bright field images were obtained on day 22 after SeV transduction.
FIG. 3 shows the results of quantitative analysis of the number of colonies in a neural colony-like form made from a different combination of indicated factors in the early stages of direct reprogramming of hiDPs (d1 to d8).
FIG. 4 shows the results of quantitative analysis of the number of colonies in a neural colony-like form made from different combinations of indicated factors in the late stages of direct reprogramming of hiDPs (d8 to d21).
FIG. 5 shows the measurement results of the expression levels of FOXA2, SHH, and LMX1A at a SHH concentration of 200 ng/mL or 800 ng/mL in a medium for direct reprogramming of hiDPs.
FIG. 6 shows a schematic diagram showing a direct reprogramming protocol for hiDPs and representative bright field images of the indicated days. EF4C means treatment with EGF, FGF2, CHIR99021, A83-01, 2-phospho-L-ascorbic acid, and NaB, whereas SF3C means treatment with SHH, FGF8, CHIR99021, A83-01, and 2-phospho-L-ascorbic acid.
FIG. 7 shows the results of confirmation by immunocytochemical staining for the expression of CORIN (which is a marker specific for the basement plate of the midbrain) in hiNSCs and hiDPs obtained in an embodiment of the present disclosure.
FIG. 8 shows the results of qRT-PCR in which the expression levels of midbrain DP-specific markers (CORIN, FOXA2, LMX1A, and EN1) in hiNSCs and hiDPs obtained in an embodiment of the present disclosure. The dCt value was calculated using the Ct value of GAPDH.
FIG. 9 shows the results of confirming the local identity of hiDPs obtained in an embodiment of the present disclosure through immunocytochemical staining for EN1 (which is a midbrain-specific marker) and HOXB1 (which is a hindbrain-specific marker).
FIG. 10 shows the counting results of the total number of cells during successive subcultures of hiDPs obtained in an example of the present invention. The data represent the logarithmic value of the fold change over the starting cell number.
FIG. 11 shows the results of confirming the presence/absence of expression of CORIN, Ki-67, PAX2, PAX5, FOXA2, LMX1A, and PAX6 in hiDPs subcultured in the middle and late stages through immunocytochemical staining.
FIG. 12 shows the results of flow cytometric analysis for CORIN and FOXA2 in hiDPs obtained in an example of the present disclosure.
FIG. 13 shows the results of immunocytochemical staining for TOM20 of hiDPs and PSC-DPs obtained in an example of the present disclosure (top). The bottom part shows the results of transforming the fluorescence images by the skeletonization function of Image J.
FIG. 14 shows the results of quantitative analysis of the number of mitochondria per cell from the skeletonized images of hiDPs and PSC-DPs obtained in an example of the present disclosure. Each dot in the box plot represents the value of one cell. The horizontal bar represents the median value. ^{∗∗} indicates P<0.01 using Student's t-test.
FIG. 15 shows the results of quantitative analysis of the number of branches per mitochondria of hiDPs and PSC-DPs obtained in an example of the present disclosure. The data represent the number of branches present in all mitochondria.
FIG. 16 shows a diagram visualizing the results of DEG analysis using a heat map.
FIG. 17 shows the results illustrating different gene expression profiles of starting cells, intermediate stage cells, and final cells through PCA plot analysis.
FIG. 18 shows the results of using DEG to perform an annotation clustering analysis between parental fibroblasts (Fb) and hiDPs obtained in an example of the present disclosure.
FIG. 19 shows the results of visualization of the gene expression level during the direct reprogramming of hiDPs by heat map analysis in the "mitotic cell cycle" GO term.
FIG. 20 shows a diagram confirming that hiDPs obtained in an embodiment of the present disclosure are highly distinguishable from Fb through scattering analysis of the transcriptome profile.
FIG. 21 shows the results of correlation analysis performed to compare the quality of hiDPs obtained in an example of the present disclosure. The transcriptome profile of DP derived from ESC according to the presence/absence of IAP classification was used as a control.
FIG. 22 shows heat map results for H3K4me3 and H3K27ac of genes analyzed during the direct reprogramming of hiDPs. The gene list represents the genes that acquire the H3K4me3 mark in the promoter region from Fb.
FIG. 23 shows a diagram illustrating the changes in gene expression levels through microarray analysis during the direct reprogramming of hiDPs. The gene list excludes genes which are not present in the microarray gene list from the gene list of FIG. 22.
FIG. 24 shows the results of analyzing the GO biological process of genes that acquired the H3K4me3 mark in the promoter region from Fb.
FIGS. 25 and 26 show the changes in the H3K4me3 and H3K27ac marks of all of the genes belonging to the GO term of "midbrain development" and "nervous system development" the direct reprogramming of hiDPs. The thick black line inside the box represents the median value. The ChIP-seq signal represents the number of reads.
FIG. 27 shows a diagram illustrating the epigenetic changes in representative midbrain dopaminergic lineage genes the direct reprogramming of hiDPs.
FIG. 28 shows heat map analysis results of H3K4me3 and H3K27ac the direct reprogramming of hiDPs. The gene list was obtained from Fb by the gene which lost the H3K4me3 mark in the promoter region.
FIG. 29 shows the analysis results of the changes in gene expression from microarray data sets the direct reprogramming of hiDPs. The gene list excludes genes which are not present in the microarray gene list from the gene list of FIG. 28.
FIG. 30 shows the analysis results of the GO biological process of a gene which has lost the H3K4me3 mark in the promoter region from Fb.
FIG. 31 shows a genome browser shot of a gene related to fibroblasts, illustrating the analysis results of the changes in H3K4me3 and H3K27ac histone marks the direct reprogramming of hiDPs.
FIG. 32 shows the results confirming the differentiation of hiDPs and hiNSCs obtained in an embodiment of the present disclosure into TH⁺ and TUJ1⁺ neurons by immunocytochemical staining.
FIG. 33 shows a graph illustrating the results of quantitative analysis of TH⁺ dopaminergic neurons.
FIG. 34 shows a graph illustrating the results of quantitative analysis of TUJ1⁺ neurons.
FIG. 35 shows the results of immunocytochemical staining for each marker at the 12th week after starting the differentiation from hiDPs into neurons.
FIG. 36 shows scanning images of the whole plate of the hiDPs differentiated into neurons after staining with an anti-GFAP antibody.
FIG. 37 shows the results of quantitative analysis of GFAP⁺ astrocytes.
FIG. 38 shows the results of immunocytochemical staining for midbrain dopaminergic neuronal markers (TH, FOXA2, NURR1, LMX1A, and EN1) in hiDP-derived neurons.
FIG. 39 shows the results of qRT-PCR performed to compare the expression levels of FOXA2, NURR1, EN1, and HOXA2 in neurons differentiated from hiNSCs and hiDPs.
FIG. 40 shows the results of confirming the purity of hiDP-neurons through immunocytochemical staining for TPH2⁺, vGLUT1⁺, GABA⁺, and CHAT⁺ neurons.
FIG. 41 shows the results of quantitative analysis of TPH2⁺, vGLUT1⁺, GABA⁺, and CHAT⁺ neurons in hiDP-derived neurons.
FIG. 42 shows the results of scattering analysis of a transcriptome profile indicating that hiDP-derived neurons are separate from hiDPs.
FIG. 43 shows the analysis results of DAVID function annotation clustering of genes 5-fold upregulated in hiDP-derived neurons compared to hiDPs.
FIG. 44 shows the results of immunocytochemical staining for mature neuronal markers (MAP2, NEUN, and SYN) and monoamine transporters (VMAT2) to confirm the maturity and specificity of hiDP-derived neurons.
FIG. 45 shows the measurement results of dopamine secretion in hiDP-derived neurons and hiNSC-derived neurons.
FIG. 46 shows phase contrast images of hiDP-derived neurons, which were cultured on a bath of a patch clamp set (left) and with a patch pipette attached to a membrane (right).
FIG. 47 shows a drawing illustrating the spontaneous activation potential (AP) of hiDP-derived neurons.
FIG. 48 shows a drawing illustrating the recording of the changes in membrane potential induced by the current injection step (current protocol: top) before (middle) and after (bottom) treatment with Na⁺ channel blocker tetrodotoxin (TTX).
FIG. 49 shows a drawing illustrating AP recording caused in response to an amount of injected current.
FIG. 50 shows a record illustrating the recoiled depolarization (arrows) triggered after AP by repetitive short hyperpolarization.
FIG. 51 shows the records of the whole-cell current against the recording of the current the inner Na⁺(INa) and outer K⁺ (IK) induced by depolarizing according to the voltage stage (protocol: top) before the presence of TTX (middle; with inner Na⁺ current) and after the presence of TTX (bottom; blocked Na⁺ current).
FIG. 52 shows gene expression levels for predictable markers and common DP markers from the hiDP and PSC-DP microarray data sets.
FIG. 53 shows the results of qRT-PCR performed for common DP markers (FOXA2, LMX1A, and CORIN).
FIG. 54 shows the results of qRT-PCR performed for predictive markers (EN1, PAX2, PAX5, PAX8, and SPRY1).
FIG. 55 shows gene expression levels for rostral and caudal genes from hiDP and PSC-DP microarray data sets.
FIG. 56 shows the results of immunocytochemical staining for the cell cycle (Ki-67) marker on the indicated days after starting the differentiation from hiDPs into neurons.
FIG. 57 shows the results of quantitative analysis of Ki-67⁺ cells of hiDP-derived neurons.
FIG. 58 shows a drawing illustrating the mutation abundance of hiDPs from Fb to the 22nd subculture during the direct reprogramming of hiDPs in all chromosomes, and representatively illustrating the results on chromosome no. 14. SNVs were identified by comparison to a gender matched reference human genome.
FIG. 59 shows the karyotype of hiDPs which were subcultured 24 times.
FIG. 60 shows a graph illustrating the number of rotations induced by apomorphine in mice induced with Parkinson's disease according to the presence/absence of hiDP transplantation.
FIG. 61 shows the results of immunocytochemical staining of grafts stained with TH and DAT. For staining purpose, each mouse was sacrificed 12 weeks after transplantation.
FIG. 62 shows the results of quantitative analysis of TH⁺ neurons in PD model mice.
FIG. 63 shows the images illustrating Nissl staining in mouse striatum to confirm graft-induced tumor formation.
FIG. 64 shows the images illustrating DAB staining for human-specific mitochondria to confirm graft-induced tumor formation.
FIG. 65 shows the results confirming the tumor-forming ability of hiDPs (n = 6) and differentiated hiDPs (5 dpd, n = 8 and 7 dpd, n = 10) by subcutaneous transplantation in immunodeficient mice. Homogenous hiPSC (n = 6) was used as a positive control.
FIG. 66 shows images of immunodeficient mice in the experimental group. In the image of the hiPSC injection group, the rest of the images were obtained at 6 wpi except the rightmost image, which is the image obtained at the 10th week. Photos of all other groups were obtained at 10 wpi. Dotted line indicates tumor.
FIG. 67 shows representative results of immunocytochemical staining of DP markers for confirming the characteristics of A-hiDPs obtained in an embodiment of the present disclosure.
FIG. 68 shows the counting results of the total number of cells during successive subcultures of A-hiDPs obtained in an example of the present disclosure. The data represent the logarithmic value of the fold change over the starting cell number.
FIG. 69 shows the results of karyotyping after long-term cultivation of A-hiDP obtained in an example of the present disclosure.
FIG. 70 shows the results of confirming the expression of CORIN and FOXA2 in A-hiDP subcultured at the late stages (p20 to p21) of A-hiDPs obtained in an example of the present disclosure by flow cytometry.
FIG. 71 shows the results of immunocytochemical staining of the cells differentiated from A-hiDPs obtained in an example of the present disclosure for midbrain dopaminergic neuronal markers.
FIG. 72 shows the result of quantitative analysis of TH⁺ neurons in the cells differentiated from A-hiDP obtained in an example of the present disclosure.
FIG. 73 shows the results of immunostaining for TPH2⁺, vGLUT1⁺, GABA⁺, and CHAT⁺ neurons in cells differentiated from A-hiDPs obtained in an example of the present disclosure.
FIG. 74 shows the results of confirming *in vitro* function by measuring dopamine secretion in hiDP-derived neurons and A-hiDP-derived neurons.
FIG. 75 shows the results of immunocytochemical staining to confirm the expression of CORIN, FOXA2, Ki-67, PAX6, and LMX1A in the PBMC-derived hiDPs obtained in an example of the present disclosure.
FIG. 76 shows the results of confirming the expression level of midbrain DP markers in the PBMC-derived hiDPs obtained in an example of the present disclosure by qRP-PCR.
FIG. 77 shows representative bright field images of the result of differentiating PBMC-derived hiDPs into neurons obtained in an example of the present disclosure.
FIG. 78 shows the results of confirming the expression levels of midbrain dopaminergic neurons and neuronal maturation markers by qRP-PCR after differentiating PBMC-derived hiDPs obtained in an embodiment of the present disclosure into neurons.
FIG. 79 shows the images, in which the direct reprogramming of hiDPs was attempted using CHIR98014 *(i.e.,* a WNT signaling agonist) and SB431542 *(i.e.,* a TGF-β inhibitor) in addition to CHIR99021 *(i.e.,* a WNT signaling agonist) and A83-01 *(i.e.,* a TGF-β inhibitor) used in an embodiment of the present disclosure, and the hiDPs separated under each condition was confirmed.
FIG. 80 shows graphs confirming the expression of major marker genes in hiDP made using CHIR98014 and SB431542. As a control, hiDPs prepared using hair fibroblasts, CHIR99021, and A83-01 was used.
FIG. 81 shows a drawing illustrating the changes in the expression of OCT4 and NANOG, which are pluripotent markers, measured by qRT-PCR during the reprogramming of hiDPs and hiPSCs.
FIG. 82 shows a drawing illustrating the changes in the expression of DP markers (EN1, LMX1A, and FOXA2) and NSC markers (PAX6) measured by qRT-PCR during the reprogramming of hiDPs and hiNSCs.
FIG. 83 shows a schematic diagram of a process of reprogramming hiPSCs and hiDPs according to the presence/absence of heat shock.
FIG. 84 shows the results confirming the hiPSCs produced under conditions in which reprogramming to hiPSCs proceeds through alkaline phosphatase staining (the top three conditions in FIG. 83).
FIG. 85 shows the results of immunocytochemical staining for FOXA2 under direct reprogramming to hiDPs (the bottom two conditions of FIG. 83).
FIG. 86 shows the results confirming the hiPSC produced under conditions in which reprogramming to hiPSCs proceeds through alkaline phosphatase staining (the second and third conditions in FIG. 83).
FIG. 87 shows a schematic diagram of an experiment for confirming whether the direct reprogramming conditions for mouse iDPs are also effective in human fibroblasts.
FIG. 88 shows the shape of the cells finally formed as a result of an experiment which was performed by applying the conditions for direct reprogramming of mouse iDPs to human fibroblasts.

### Best Mode for Carrying out the Invention

Hereinafter, the present disclosure will be explained in detail.

### Method for preparing induced dopaminergic neuronal progenitors

In one aspect, the present disclosure relates to a method for preparing induced dopaminergic neuronal progenitors (iDPs), which comprises a) introducing one or more genes selected from the group consisting of Oct4, Sox2, Klf4, and Myc into adult cells; b) culturing the cells in a medium containing EGF and FGF2; and c) culturing the cells in a medium containing FGF8, SHH, a Wnt signaling agonist, and a TGF-β inhibitor.

As used herein, the term "adult cell" refers to a cell in which differentiation has occurred, and refers to a cell in a state in which the pluripotency, which refers to the ability to differentiate into various types of cells, is completely lost or mostly lost. In the present disclosure, it may refer to a cell in which the differentiation has been completed, and may refer to a cell that becomes a target capable of recovering some pluripotency or totipotency by increasing the expression level of a pluripotent factor.

Specifically, the adult cells include fibroblasts, peripheral blood mononuclear cells (PBMCs), mesenchymal stem cells (MSCs), *etc.,* but are not limited thereto.

In addition, the adult cells may be derived from humans.

In one embodiment of the present disclosure, for human neonatal fibroblasts, human adult fibroblasts, and human peripheral blood mononuclear cells, human iDPs (hiDPs) can be successfully obtained by applying the method for preparing iDPs of the present disclosure.

As used herein, the expression "one or more genes selected from the group consisting of octamer-binding transcription factor 4 (Oct4), sex determining region Y (SRY)-box 2 Sox2, Kruppel-like factor 4 (Klf4), and Myc", which are OSKM factors (Yamanaka factors) well known as reprogramming factors, refers to factors that play an important role in the process of de-differentiating differentiated cells to obtain pluripotency once again. That is, the expression refers to factors that function to maintain or acquire a cell's self-renewal ability or pluripotency. In particular, these factors may play a role in reprogramming cells, which have already undergone differentiation, into totipotent or pluripotent cells. In addition, the Myc may be L-Myc or c-Myc.

In one embodiment of the present disclosure, Oct4, Sox2, Klf4, and c-Myc may be introduced into adult cells. The introduction of the reprogramming factor may be performed by a method known in the art, for example, a Sendai virus vector may be used.

As used herein, the term "epidermal growth factor (EGF)", which is an epidermal growth factor, refers to one of the peptides that promote the proliferation of epithelial cells.

As used herein, the term "fibroblast growth factor 2 (FGF2)" refers to fibroblast growth factor 2, and is known to play an important role in proliferation and angiogenesis of endothelial cells or smooth muscle cells by stimulating fibroblasts.

As used herein, the term the term "fibroblast growth factor 8 (FGF8)" refers to fibroblast growth factor 8, which is a growth factor that stimulates fibroblasts to induce proliferation, and which is known to play an important role in the development of fetal cranial nerves.

As used herein, the term "sonic hedgehog (SHH)" refers to a protein constituting a mammalian signaling pathway called hedgehog, which is the most studied ligand in the hedgehog signaling pathway, and it is known to play an important role in regulating organ formation in vertebrates.

As used herein, the term "Wnt signaling agonist" refers to a material which activates signaling in the Wnt signaling pathway. There are at least three types of intracellular signaling pathways that are activated by the binding between Wnt and receptors (*i*.*e*., the β-catenin pathway, the planar cell polarity pathway, and the Ca²⁺ pathway).

In the β-catenin pathway, the expression of various target genes is regulated by regulating the stability of β-catenin. This pathway regulates cell proliferation or differentiation, and the genetic abnormalities of proteins constituting this pathway appear at high frequencies in human cancer. In the planar cell polarity pathway, the low molecular weight G protein Rho family is interposed and thereby activates Jun kinase or Rho kinase. In the Ca²⁺ pathway, intracellular Ca²⁺ mobilization protein is interposed and thereby activates phosphorylation enzyme C or carmodulin phosphorylation enzyme. The planar cell polarity pathway and the Ca²⁺ pathway regulate polarity or movement of cells. Wnt, which regulates the activation of these signaling pathways, is known to regulate several cellular responses.

The Wnt signaling agonist may be one or more compounds selected from the group consisting of the following, but is not limited thereto:
1) 19 kinds of Wnt proteins: Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16b;
2) materials which increase β-catenin: most cells respond to Wnt signaling by the increase of β-catenin;
3) materials which phosphorylate Dishevelled: when Wnt and frizzled (a receptor of Wnt) bind, it results in phosphorylation to thereby activate β-catenin or activate Rho or Ras in the planar cell polarity pathway;
4) inhibitors of glycogen synthase kinase 3 (GSK3): lithium (Li), LiCl, bivalent Zn, 6-bromoindirubin-3'-oxime (BIO), SB216763, SB415286, CHIR99021, CHIR98014, QS11 hydrates, TWS119, Kenpaullone, alsterpaullone, indirubin-3'-oxime, TDZD-8, Ro 31-8220 methanesulfonate (Ro 31-8220 methanesulfonate salt), *etc.;*
5) inhibitors of negative regulators of the Wnt signaling pathway (e.g., Axin, APC, *etc.*), RNAi, *etc.;*
6) proteins that activate the Wnt signaling pathway: Norrin binds to frizzled 4, and R-spondin 2 reacts with frizzled 8 and LRP6; and
7) Wnt overexpression constructs or beta-catenin overexpression constructs by gene transfer including transfection, *etc.* may be used.

In one embodiment of the present disclosure, the Wnt signaling agonist is CHIR99021 represented by the following Formula I:

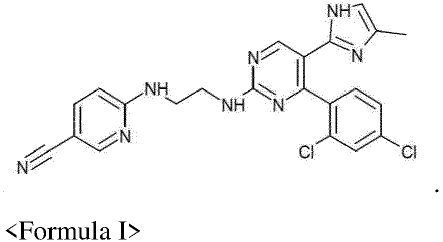

In another embodiment of the present disclosure, the Wnt signaling agonist is CHIR98014 represented by Formula II below:

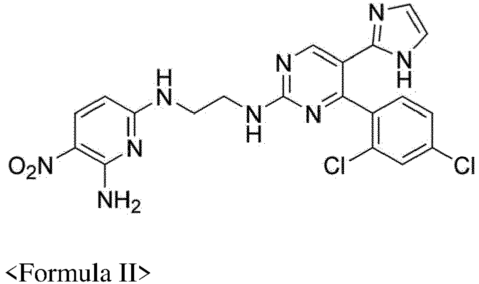

The TGF-β inhibitor may be one or more compounds selected from the group consisting of A83-01, SB431542, RepSox, LY364947, and SB525334, but is not limited thereto.

In one embodiment of the present disclosure, the TGF-β inhibitor is A83-01 represented by Formula III below:

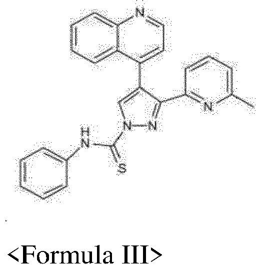

In another embodiment of the present disclosure, the TGF-β inhibitor is SB431542 represented by Formula IV below:

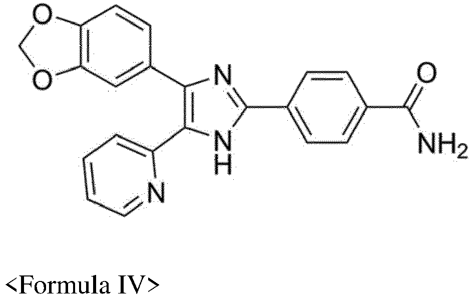

In the present disclosure, after the cultivation of Step b), a step of further culturing by adding Y-27632 (which is a Rho-associated protein kinase (ROCK) inhibitor) may be further comprised. The additional culture may be performed for 12 to 36 hours, and specifically for 24 hours.

In the present disclosure, the medium of Step b) may further comprise one or more compounds selected from the group consisting of a Wnt signaling agent, a TGF-β inhibitor, 2-phospho-L-ascorbic acid, and sodium butyrate (NaB). Specifically, the medium of Step b) may further comprise a Wnt signaling agonist and a TGF-β inhibitor.

In the present disclosure, the medium of Step c) may further comprise 2-phospho-L-ascorbic acid.

In the present disclosure, the medium of Step b) may comprise 1 ng/mL to 100 ng/mL of EGF and 1 ng/mL to 100 ng/mL of FGF2.

In one embodiment of the present disclosure, the medium of Step b) may comprise 20 ng/mL of EGF, 20 ng/mL of FGF2, 3.0 µM of CHIR9902, 0.5 µM of A83-01, 50 ng/mL of 2-phospho-L-ascorbic acid, and 0.2 mM of NaB. As a basal medium, human neuron reprogramming medium (RepM-Neural) may be used.

In the present disclosure, the medium of Step c) may comprise 10 ng/mL to 1,000 ng/mL of FGF8, 100 ng/mL to 2,000 ng/mL of SHH, 0.1 µM to 50.0 µM of Wnt signaling agonist, and 0.01 µM to 10.0 µM of a TGF-β inhibitor.

In one embodiment of the present disclosure, the medium of Step c) may comprise 100 ng/mL of FGF8, 800 ng/mL of SHH, 3.0 µM of CHIR9902, 0.5 µM of A83-01, and 50 ng/mL of 2-phospho-L-ascorbic acid. As a basal medium, RepM-Neural may be used.

In the present disclosure, Step a) may be performed for 12 to 36 hours, specifically for 24 hours.

In the present disclosure, Step b) may be performed for 5 to 9 days, specifically for 7 days.

In one embodiment of the present disclosure, Step b) may be performed such that the cultivation is performed for 7 days in a medium including EGF, FGF2, CHIR99021, and A83-01, and then Y-27632 is further added to the same medium and cultured for additional 24 hours.

In the present disclosure, Step c) may be performed for 10 to 18 days, specifically 13 to 15 days.

In the present disclosure, the preparation method may directly reprogram iDPs from adult cells. Specifically, the preparation method does not undergo a step of preparing a pluripotent intermediate from adult cells.

In one embodiment of the present disclosure, as a result of measuring the changes in expression levels of pluripotent cell-specific markers, iNSCs-specific markers, and midbrain basal plate-specific markers, it can be confirmed that the hiDPs reprogramming pathway is separate from the hiPSCs and hiNSCs reprogramming pathways, and it can be confirmed that the hiDPs of the present disclosure are directly produced from fibroblasts without undergoing the pluripotent intermediate step by way of performing the hiPSC and hiDPs reprogramming process according to the presence/absence of a heat shock step.

### Induced dopaminergic neuronal progenitors

In another aspect, the present disclosure relates to induced dopaminergic neuron precursors (iDPs) prepared by the preparation method described above.

In another aspect of the present disclosure, the present disclosure relates to an induced dopaminergic neuronal progenitor, in which (i) one or more genes selected from the group consisting of CORIN, FOXA2, and LMX1A exhibit reduced expression compared to dopaminergic neuronal progenitors derived from pluripotent stem cells; (ii) one or more genes selected from the group consisting of EN1, PAX2, PAX5, PAX8, and SPRY1 exhibit increased expression compared to dopaminergic neuronal progenitors derived from pluripotent stem cells; or (iii) the reduced expression of (i) and the increased expression of (ii) are exhibited.

In one embodiment of the present disclosure, as a result of comparing the relative expression levels of well-known dopaminergic neuron-specific markers (FOXA2, LMX1A, and CORIN) based on the expression level of the iDPs, it was confirmed that the expression levels of the iDPs were 1,000- to 100,000-fold lower than those of the PSC-DPs. In addition, as a result of comparing the relative expression levels of predictive markers (EN1, PAX2, PAX5, PAX8, and SPRY1) for transplantation results based on the expression level of PSC-DP, it was confirmed that the expression levels of the iDPs were 3- to 300-fold higher than those of the PSC-DPs.

In the induced dopaminergic neuron precursors, one or more genes selected from the group consisting of CORIN, FOXA2, and LMX1A can exhibit 1,000-fold to 100,000-fold reduced expression compared to the dopaminergic neuron precursors derived from pluripotent stem cells. Specifically, LMX1A can exhibit reduced expression of 1,000-to 100,000-fold, 10,000- to 100,000-fold, 10,000- to 90,000-fold, 10,000- to 80,000-fold, 10,000- to 70,000-fold, 10,000- to 60,000-fold, 10,000- to 50,000-fold, 10,000- to 40,000-fold, 10,000- to 30,000-fold, or 10,000- to 20,000-fold; and CORIN and FOXA2 can exhibit reduced expression of 1,000- to 100,000-fold, 1,000- to 10,000-fold, 1,000- to 9,000-fold, 1,000- to 8,000-fold, 1,000- to 7,000-fold, 1,000- to 6,000-fold, 1,000- to 5,000-fold, 1,000- to 4,000-fold, 1,000- to 3,000-fold, or 1,000- to 2,000-fold. More specifically, FOXA2 can exhibit reduced expression of 1,000- to 10,000-fold, 2,000- to 9,000-fold, 3,000- to 8,000-fold, 4,000- to 7,000-fold, or 5,000- to 6,000-fold; LMX1A can exhibit reduced expression of 10,000- to 100,000 fold, 20,000- to 90,000-fold, 30,000- to 80,000-fold, 30,000- to 70,000-fold, or 35,000- to 65,000-fold; CORIN can exhibit reduced expression of a 1,000- to 5,000-fold, 1,200- to 4,500-fold, 1,500- to 4,000-fold, 1,700- to 3,500-fold, or 2,000- to 3,000-fold.

In the induced dopaminergic neuron precursors, one or more genes selected from the group consisting of EN1, PAX2, PAX5, PAX8, and SPRY1 can exhibit a 3- to 300-fold increase in expression compared to the dopaminergic neuron precursor derived from pluripotent stem cells; specifically, increased expression of 3- to 300 times, 3- to 200-fold, 3- to 100-fold, 3- to 90-fold, 3- to 80-fold, 3- to 70-fold, 3- to 60-fold, 3- to 50-fold , 3- to 40-fold, 3- to 30-fold, 3- to 20-fold, 3- to 10-fold; and more specifically, increased expression of 3- to 300-fold, 3- to 270-fold, 3- to 260-fold, 3- to 250-fold, 3- to 240-fold, 3- to 230-fold, or 3- to 220-fold.

The induced dopaminergic neuron precursors may not express HOXB1. In one embodiment of the present disclosure, the iDPs express CORIN (a dopaminergic neuronal precursor-specific marker) and FOXA2, LMX1A, and EN1 (midbrain base plate-specific markers), and may not express HOXB1 (a hindbrain-specific marker). From these results, it can be seen that the iDPs of the present disclosure have highly pure midbrain-specific properties.

In addition, the iDPs may express LMX1A at the mRNA level, but may not express LMX1A at the protein level. In addition, dopaminergic neurons differentiated from the induced dopaminergic neuronal precursors can express LMX1A not only at the mRNA level but also at the protein level.

From these results, it can be seen that the iDPs of the present disclosure are separate cells distinguishable from PSC-DPs, which are known to be implantable *in vivo* for the treatment of Parkinson's disease, and are more suitable for PD treatment through *in vivo* transplantation.

In addition, the iDPs may show reduced expression of endogenous Oct4 and NANOG compared to iPSCs; may show reduced expression of PAX6 compared to iNSCs; and may show increased expression of EN1, LMX1A, and FOXA2 compared to iNSCs.

In one embodiment of the present disclosure, it can be seen that the iDPs exhibit reduced expression of endogenous Oct4 and NANOG (which are pluripotent cell-specific markers) compared to iPSCs; and reduced expression of PAX6 (which is an iNSCs-specific marker) compared to iNSCs, and increased expression of EN1, LMX1A, and FOXA2 (which are midbrain base plate-specific markers) compared to iNSCs.

From these results, it can be seen that the hiDPs reprogramming process is separate from the hiPSCs and hiNSCs reprogramming pathways.

### Pharmaceutical composition containing induced dopaminergic neuronal progenitors

In another aspect, the present disclosure relates to a pharmaceutical composition for preventing or treating Parkinson's disease, which contains the induced dopaminergic neuron precursor as an active ingredient.

As used herein, the term "Parkinson's Disease (PD)" is a disease caused by the gradual loss of dopaminergic neurons distributed in *substania nigra* of the brain, and is a chronic progressive degenerative disease of the nervous system. It is estimated that patients with Parkinson's disease account for about 1% of the population in people 60 years of age and older. The cause of Parkinson's disease has not yet been elucidated, but according to a general theory, it is a multifactorial disease such as genetic factors, mutation-induced factors, protein dysfunction, *etc.* Although the exact cause has not been identified, it is common that symptoms due to the loss of dopaminergic neurons in the midbrain occur. Therefore, Parkinson's disease is being treated by preventing the loss of the dopaminergic neurons, replacing the dopaminergic neurons, alleviating the symptoms caused by the loss of dopaminergic neurons, *etc.*

In one embodiment of the present disclosure, a remarkable improvement of motor defects can be seen from the 4th week after transplanting the hiDPs differentiated into neurons for more than 10 days in a PD mouse model.

From these results, it can be seen that the hiDPs of the present disclosure implanted *in vivo* differentiate into functional dopaminergic neurons, which are highly likely to contribute to recovery of exercise in a PD mouse model.

The pharmaceutical composition of the present disclosure may contain conventional and non-toxic pharmaceutically acceptable additives prepared into a formulation according to a conventional method. For example, the pharmaceutical composition may further contain a pharmaceutically acceptable carrier, diluent or excipient.

Examples of additives used in the composition of the present disclosure include sweeteners, binders, solvents, dissolution aids, wetting agents, emulsifiers, isotonic agents, absorbents, disintegrants, antioxidants, preservatives, lubricants, glidants, fillers, flavoring agents, *etc.* For example, the additives may include lactose, dextrose, sucrose, mannitol, sorbitol, cellulose, glycine, silica, talc, stearic acid, stearin, magnesium stearate, magnesium aluminosilicate, starch, gelatin, gum tragacanth, alginic acid, sodium alginate, methylcellulose, sodium carboxymethylcellulose, agar, water, ethanol, polyethylene glycol, polyvinylpyrrolidone, sodium chloride, calcium chloride, *etc.*

The composition of the present disclosure may be prepared in various formulations for parenteral administration (e.g., intravenous, intramuscular, subcutaneous, or intracranial administration). In particular, the composition of the present disclosure may be administered intracranially (e.g., intracerebrovascular, intrathecal, or intracerebroventricular administration). Specifically, the composition of the present disclosure may be administered by lateral cerebro ventricular injection into the brain of an individual. For example, the injection may be performed through an intraventricular catheter system, which includes a burrhole and a cisternal prepared in the subject's skull, or a reservoir implanted in the subject's skull, and a catheter connected to the reservoir.

Specifically, preparations for parenteral administration include sterilized aqueous solutions, non-aqueous solutions, suspensions, emulsions, lyophilized preparations, and suppositories. As the non-aqueous solvent and suspending agent, propylene glycol, polyethylene glycol, vegetable oil (*e.g.*, olive oil), an injectable ester (*e.g.*, ethyl oleate), *etc.* may be used. As the base of suppositories, Withepsol, Macrogol, Tween61, cacao butter, laurinum, glycerogelatin, *etc.* may be used. Meanwhile, the injection may contain conventional additives (e.g., solubilizing agents, isotonic agents, suspending agents, emulsifying agents, stabilizing agents, preservatives, *etc*.).

The composition of the present disclosure may be administered to a patient in a therapeutically effective amount or pharmaceutically effective amount.

In particular, the term "therapeutically effective amount" or "pharmaceutically effective amount" refers to an amount of a compound or composition which is effective to prevent or treat the subject disease, which is sufficient to treat the disease at a reasonable benefit/risk ratio applicable to medical treatment, and an amount which does not cause side effects. The level of the effective amount can be determined depending on factors including the health condition of the patient, the type of disease, the severity, the activity of the drug, the sensitivity to the drug, the method of administration, the time of administration, the route of administration and the rate of excretion, the duration of treatment, the drugs used in combination or concurrently, and other factors well known in the medical field.

The composition of the present disclosure may be administered as an individual therapeutic agent or administered in combination with other therapeutic agents, may be administered sequentially or simultaneously with a conventional therapeutic agent, and may be administered once or multiple times. It is important to administer an amount capable of obtaining the maximum effect in a minimum amount without side effects in consideration of all the factors described above, and this can easily be determined by a person skilled in the art.

Specifically, the effective amount of the compound in the composition of the present disclosure may vary depending on the age, sex, and weight of the patient, and generally, it can be administered about 0.1 mg to about 1,000 mg, or about 5 mg to about 200 mg per kg of body weight daily or every other day or divided into 1 to 3 times daily. However, since the effective amount may increase or decrease depending on the route of administration, the severity of the disease, sex, weight, age, *etc.,* the scope of the present disclosure is not limited thereto.

### Method for preventing or treating Parkinson's disease

In another aspect, the present disclosure relates to a method for preventing or treating Parkinson's disease, which comprises administering the pharmaceutical composition to an individual in a therapeutically effective amount.

In still another aspect, the present disclosure relates to a use of the induced dopaminergic neuron precursor for preventing or treating Parkinson's disease.

In still another aspect, the present disclosure relates to a use of the induced dopaminergic neuron precursor for the preparation of a medicament for preventing or treating Parkinson's disease.

### Method for screening agents for preventing or treating Parkinson's disease

In still another aspect, the present disclosure relates to a method for screening agents for preventing or treating Parkinson's disease, which comprises treating the induced dopaminergic neuronal progenitor with a candidate material for preventing or treating Parkinson's disease; and measuring the proliferation ability, activity, or differentiation ability into dopaminergic neurons of the induced dopaminergic neuronal progenitors, compared to the control group not treated with the candidate material

In still another aspect, the present disclosure relates to a composition for screening agents for preventing or treating Parkinson's disease containing the induced dopaminergic neuron precursor.

In still another aspect, the present disclosure relates to a use of the induced dopaminergic neuron precursor for screening agents for preventing or treating Parkinson's disease.

As used herein, the term "candidate material as an agent for preventing or treating Parkinson's disease" may refer to an individual nucleic acid, protein, other extract or natural product, compound, *etc.,* which are presumed to have the possibility of preventing or treating Parkinson's disease according to a conventional selection method, selected randomly.

As used herein, the term "control group", which is a group containing induced dopaminergic neuron precursors not treated with a candidate material for preventing or treating Parkinson's disease, refers to a group containing cells belonging to a parallel relationship with the group treated with the candidate material.

In the present disclosure, the method for screening agents for preventing or treating Parkinson's disease may be designed in such a manner that a candidate materials are treated with the induced dopaminergic neuron precursor of the present disclosure and compared with a control group not treated with the candidate material.

In the present disclosure, in the case where the induced dopaminergic neuron precursors are treated with a candidate material for preventing or treating Parkinson's disease, if the proliferative power or activity of the iDPs is increased or the ability of the iDPs to differentiate into neurons is increased, a step of determining the candidate material as an agent for preventing or treating Parkinson's disease may be further comprised.

The material selected by such a screening method acts as a leading compound in the subsequent development of Parkinson's disease prevention or treatment, and by modifying and optimizing the leading material, a new agent for preventing or treating Parkinson's disease can be developed.

### Mixture or medium composition for preparing induced dopaminergic neuron precursors

In still another aspect, the present disclosure relates to a mixture for preparing an induced dopaminergic neuron precursor, which contains adult cells into which one or more genes selected from the group consisting of Oct4, Sox2, Klf4, and Myc are introduced; EGF; and FGF2.

The mixture for preparing the induced dopaminergic neuron precursor may further comprise a Wnt signaling agonist and a TGF-β inhibitor.

Description of the induced dopaminergic neuron precursor, the Wnt signaling agonist, and the TGF-β inhibitor are the same as described above.

In still another aspect, the present disclosure relates to a mixture, which contains an induced dopaminergic neuron precursor, FGF8, SHH, Wnt signaling agonist, and a TGF-β inhibitor.

In still another aspect, the present disclosure relates to a medium composition for preparing an induced dopaminergic precursor, which contains an EGF, FGF2, a Wnt signaling agonist, and a TGF-β inhibitor.

In still another aspect, the present disclosure relates to a medium composition for preparing or maintaining an induced dopaminergic precursor, which contains an FGF8, SHH, a Wnt signaling agonist, and a TGF-β inhibitor.

In one embodiment of the present disclosure, it can be seen that in the process of directly reprogramming hiDPs from adult cells, EGF and FGF2 are essential in the early stage, and simultaneous treatment of SHH, FGF8, a Wnt signaling agonist, and a TGF-β inhibitor is the most efficient in the later stage.

Hereinafter, the present disclosure will be described in more detail through examples. These examples are for illustrative purposes only, and the contents of the present disclosure are not limited by these examples.

### Example 1. Direct reprogramming of hiDPs

### Example 1.1. Development of direct reprogramming protocol for hiDPs

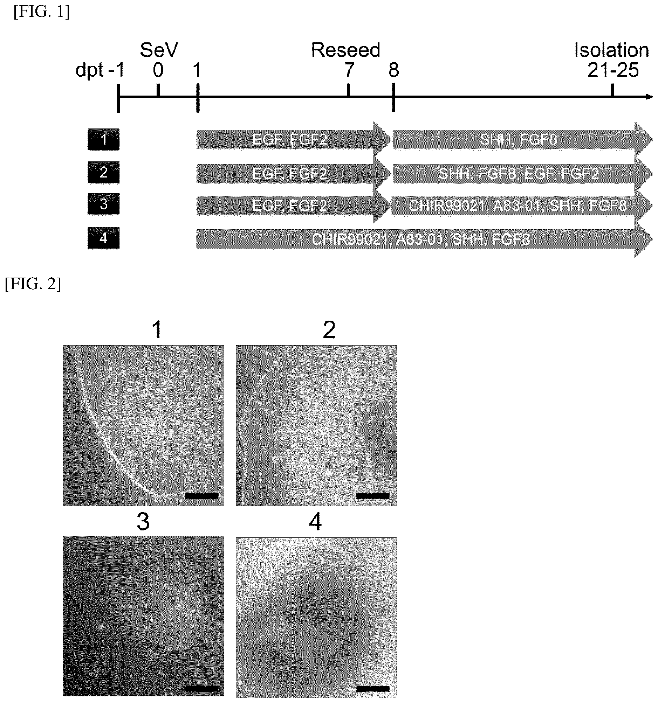

Based on previous studies in a mouse model (Korean Patent Publication No. 10-2015-0015294), in order to develop a protocol for preparing a human induced dopaminergic neuron precursor, direct reprogramming of hiDPs was performed by combining several factors along with the induction of expression of the OSKM (Oct4, Sox2, Klf4, c-Myc) factors (Conditions 1 to 4 of FIG. 1).

In the case of Condition 4 in which the treatment of EGF and FGF2 was not comprised, there was no formation of cell colonies (FIG. 2). Therefore, it was confirmed that the treatment of EGF and FGF2 is essential for the early stage of direct reprogramming of hiDPs.

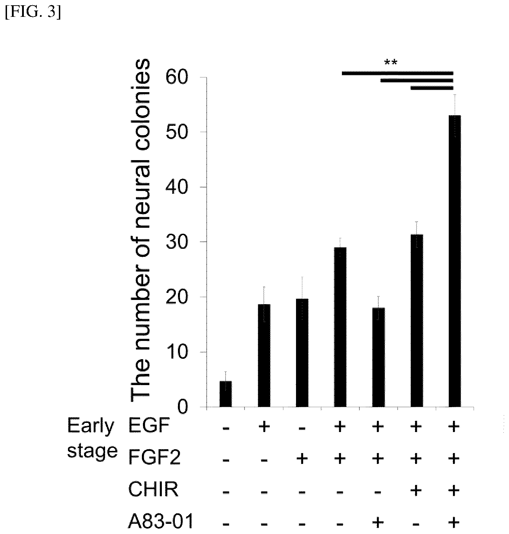

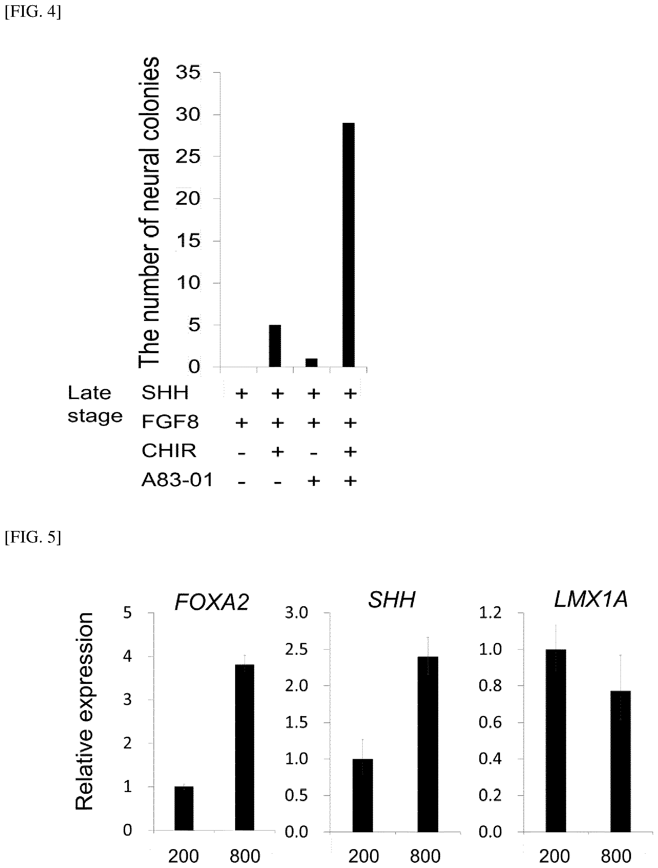

In addition, in order to establish a direct reprogramming protocol for hiDPs, a combination of several factors in the early and late stages of direct reprogramming was tested. As a result, in the early stage, it was confirmed that the number of colonies was the largest when a combination of CHIR99021 (which is a WNT signaling agonist) and A83-01 (which is_a TGF-β inhibitor) (hereinafter collectively referred to as CHA) together with EGF and FGF2 was used (FIG. 3). Meanwhile, it was also required that after EGF, FGF2, CHIR99021, and A83-01 were treated in the initial stage, CHA treatment was also necessary in the late stage, and that when SHH and FGF8 were treated together, a significantly increased number of colonies was formed (FIG. 4).

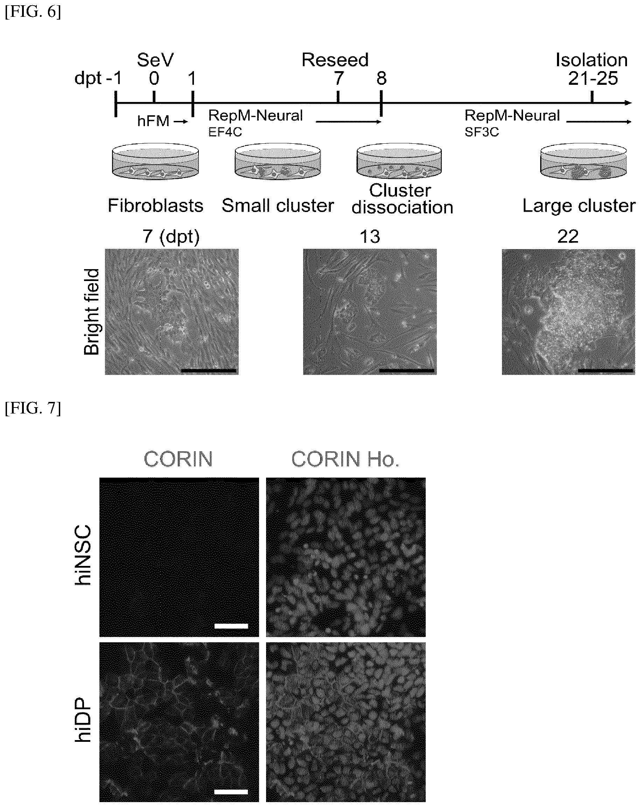

Since SHH signaling increases FOXA2 expression in midbrain development, it increased the concentration of SHH up to 800 ng/mL, which up-regulated the expression levels of FOXA2 and SHH, but there was no effect on the expression level of LMX1A (FIG. 5). Through these processes, the protocol for direct reprogramming of hiDPs was established (FIG. 6).

### Example 1.2. Direct reprogramming of hiDPs from human neonatal fibroblasts

Human neonatal fibroblasts (CRL-2097) were purchased from the American Type Culture Collection (Rockville, MD, USA), and CRL-2097 was maintained in an hFM medium (a modified Eagle's medium, Thermo Fisher Scientific, Waltham, MA, USA) supplemented with (10% fetal bovine serum (FBS) and 0.1 mM non-essential amino acids).

CRL-2097 was plated at a density of 30,000 cells/well in a 24-well plate. The next day (day 0 after transduction, 0 dpt), the cells were transduced with OKSM factors with the ploidy of transduction suitable for the cells (MOI; KOS:M:K = 4.2:4.2:2.5) using a Sendai virus (SeV) mixture (CytoTune^{™}-iPS 2.0 Sendai reprogramming kit; Thermo Fisher Scientific).

After culturing the transduced CRL-2097 for 24 hours, the medium containing the SeV mixture was replaced, for a week, with a human neuron reprogramming medium (which was supplemented with RepM-Neural: 0.05% Albumax-I, IX N2, IX B27 minus vitamin A, 2 mM Glutamax, and 0.11 mM β-mercaptoethanol, and in which Advanced DMEM/F12 and a Neurobasal medium were mixed at a 1:1 ratio; all purchased from Thermo Fisher Scientific), which contained 3.0 µM CHIR99021 (Tocris), 0.5 µM A83-01 (Tocris), 50 µg/mL 2-phospho-L-ascorbic acid. (Sigma-Aldrich, St. Louis, MO, USA), 0.2 mM NaB (Sigma-Aldrich), 20 ng/mL epithelial growth factor (EGF; Peprotech), and 20 ng/mL fibroblast growth factor 2 (FGF2; Peprotech).

After a week, the cultured cells were dissociated with Accutase (Millipore) with 10 µM Y-27632 (Tocris), and replated with the same medium containing 10 µM Y-27632 on Geltrex-coated 6-well plates at 7 dpt.

On the next day, the culture medium was replaced with RepM-Neural, in which 3.0 µM CHIR99021, 0.5 µM A83-01, 50 µg/mL 2-phospho-L-ascorbic acid, 100 ng/mL FGF8 (Peprotech), and 800 ng/mL Sonic Hedgehog (SHH; R&D systems, Minneapolis, MN, USA) were further added. After 13 to 15 days, some colonies were isolated to obtain hiDPs, and the hiDPs were maintained on Geltrex-coated plates using a medium with reduced SHH concentration (200 ng/mL).

### Example 1.3. Direct reprogramming of hiDPs from adult human fibroblasts

Human adult fibroblasts (HDF4) were obtained from National Chungnam National University Hospital (Daejeon, Korea), and were sujbected to direct reprogramming of hiDPs in the same manner as described in Example 1.2, and adult somatic cell-derived hiDPs (A-hiDPs) were obtained therefrom.

### Example 1.4. Direct reprogramming of hiDPs from human peripheral blood mononuclear cells

Human peripheral blood mononuclear cells (PBMC; PBMNC015C) were purchased from StemExpress (Folsom, CA, USA).

30,000 PBMCs were transduced by mixing with a SeV mixture (CytoTune^{™}) with a suitable transduction ploidy (MOI; KOS:M:K = 4.2:4.2:2.5) and centrifuging at 2,250 rpm for 90 minutes at room temperature. The supernatant of the transduced PBMC was removed, transferred to a culture dish coated with iMatrix511, and cultured in an incubator at 37°C for 24 hours.

After incubation, the resultant was centrifuged at 2,250 rpm for 10 minutes at room temperature and the supernatant was removed. After dissociating the cells, the cells were cultured, for 14 days, in a human neuron reprogramming medium (which was supplemented with RepM-Neural: 0.05% Albumax-I, IX N2, IX B27 minus vitamin A, 2 mM Glutamax, and 0.11 mM β-mercaptoethanol, and in which Advanced DMEM/F12 and a Neurobasal medium were mixed at a 1:1 ratio), which contained 3.0 µM CHIR99021, 0.5 µM A83-01, 50 µg/mL 2-phospho-L-ascorbic acid, 0.2 mM NaB, 20 ng/mL EGF, and 20 ng/mL FGF2.

After 14 days, the cultured cells were dissociated with Accutase with 10 µM Y-27632 and replated with the same medium containing 10 µM Y-27632 on iMatrix511 (Nippi)-coated 6-well plates.

On the next day, culture medium was replaced with RepM-Neural, in which 3.0 µM CHIR99021, 0.5 µM A83-01, 50 µg/mL 2-phospho-L-ascorbic acid, 100 ng/mL FGF8, and 800 ng/mL SHH were further added. After 13 to 15 days, some colonies were isolated to obtain PBMC-hiDPs (70262-01 and 70262-02), and the hiDPs were maintained on iMatrix511-coated plates using a medium with reduced SHH concentration (200 ng/mL).

### Comparative Example 1. hiNSC Reprogramming

For reprogramming into human induced neural stem cells (hiNSCs), human fibroblasts (CRL-2097) were plated at a density of 30,000 cells/well on 24-well plates. On the next day, a SeV mixture (CytoTune^{™}) was transduced with the ploidy of transduction suitable for the cells (MOI; KOS:M:K = 4.2:4.2:2.5).

After culturing the transduced CRL-2097 for 24 hours, the medium containing the SeV mixture was replaced with a human neuronal reprogramming medium containing 3.0 µM CHIR99021, 0.5 µM A83-01, and 10 ng/mL hLIF (Peprotech). The medium was replaced every other day. At 7 dpt, the cultured cells were dissociated with Accutase and replated. At 21 dpt, some neural colonies were isolated to obtain hiNSCs, and the hiNSCs were maintained on Geltrex-coated plates using the same medium.

### Comparative Example 2. hiPSC Reprogramming

For reprogramming into hiPSCs, human fibroblasts (CRL-2097) were plated at a density of 30,000 cells/well on 24-well plates. On the next day, the cells were transduced with a SeV mixture (CytoTune^{™}) according to the manufacturer's instructions.

After incubating the transduced CRL-2097 for 24 hours, the medium containing the SeV mixture was replaced with hFM. At 3 dpt, the culture medium was replaced with mTeSR-1 medium (Stemcell Technologies) containing 1 mM nicotinamide (Sigma-Aldrich). At 7 dpt, the cells were dissociated with Accutase and replated on Geltrex-coated 6-well plates. At 21 dpt, some colonies were isolated to obtain hiPSCs, and the hiNSCs were maintained on Geltrex-coated plates using the same medium.

### Comparative Example 3. Preparation of PSC-DP

hiPSCs were plated at a density of 500,000 cells/well on iMatrix 511-coated 24-well plates. The next day (day 0), GMEM supplemented with 8% Knockout Serum Replacement, 0.1 mM MEM NEAA, 0.1 mM sodium pyruvate, and 0.1 mM 2-mercaptoethanol (all purchased from Thermo Fisher Scientific) were further added with 100 nM LDN193189 and 0.5 uM A83-01 for 8 days to induce neural induction. Then, 100 ng/mL FGF8, 2 µM purmorphamine, and 100 ng/mL SHH were added thereto for 7 days (neural induction: day 1 to day 7), and cultured during day 3 to day 12 by adding 3.0 uM CHIR99021 and 0.2 mM ascorbic acid thereto for nerve induction, and thereby PSC-DPs were obtained.

### Experimental protocols

Experimental protocols used to characterize the cells obtained in Example 1 and Comparative Examples 1 to 3 are described in Examples 2 to 8 below.

### Example 2. RNA isolation and quantitative reverse transcription-polymerase chain reaction (qRT-PCR)

Total RNA was extracted from the cells using an RNeasy mini kit containing QiaShredder (Qiagen, Hilden, Germany) and DNase I (Qiagen). The extracted RNA was reverse transcribed using an iScript cDNA synthesis kit (Bio-Rad, Hercules, CA, USA). In one qPCR reaction, a 1/50 dilution of the synthesized cDNA template was used, and a mixture prepared by adding iQ^{™} SYBR Green Supermix (Bio-rad) and primers thereto was subjected to qRT-PCR using the Applied Biosystems 7500 Fast Real-Time PCR System (Thermo Fisher Scientific). The primer sequences used in qRT-PCR are shown in Table 1 below.

**[Table 1]**

| **Gene** | **Forward primer** | **SEQ ID NO** | **Reverse primer** | **SEQ ID NO** |
|---|---|---|---|---|
| *FOXA2* | GGAGCAGCTACTATGCAGA GC | 1 | CGTGTTCATGCCGTTCATCC | 2 |
| *SHH* | CTCGCTGCTGGTATGCTCG | 3 | ATCGCTCGGAGTTTCTGGAG A | 4 |
| *LMX1A* | ACGTCCGAGAACCATCTTG AC | 5 | CACCACCGTTTGTCTGAGC | 6 |
| *CORIN* | CCAAAGCCGGTCTTGAGAG | 7 | GAGGAGGTTAGCAGTCGCC | 8 |
| *EN1* | CGCAGCAGCCTCTCGTATG | 9 | CCTGGAACTCCGCCTTGAG | 10 |
| *PAX2* | ACGAGGCTTGGAGATTCAG CAA | 11 | TGTCGGCCTGAAGCTTGATG T | 12 |
| *PAX5* | ACTTGCTCATCAAGGTGTC AG | 13 | TCCTCCAATTACCCCAGGCT T | 14 |
| *PAX8* | ATAGCTGCCGACTAAGCAT TGA | 15 | ATCCGTGCGAAGGTGCTTT | 16 |
| *SPRY1* | GCCCTGGATAAGGAACAG CTAC | 17 | GCCGAAATGCCTAATGCAAA GA | 18 |
| *Endo-OCT4* | AGTTTGTGCCAGGGTTTTT G | 19 | ACTTCACCTTCCCTCCAACC | 20 |
| *NANOG* | AACGTTCTGCTGGACTGAG C | 21 | ATGCTTCAAAGCAAGGCAAG | 22 |
| *PAX6* | GTCCATCTTTGCTTGGGAA A | 23 | TAGCCAGGTTGCGAAGAACT | 24 |
| *NURR1* | ACCACTCTTCGGGAGAATA CA | 25 | GGCATTTGGTACAAGCAAGG T | 26 |
| *HOXA2* | CCCCTGTCGCTGATACATT | 27 | TGGTCTGCTCAAAAGGAGGA | 28 |
| | TC | | G | |
| *GAPDH* | TGCACCACCAACTGCTTAG C | 29 | GGCATGGACTGTGGTCATGA G | 30 |

### Example 3. Immunocytochemical analysis

Samples to be used for analysis were fixed with 4% paraformaldehyde (Electron Microscopy Sciences, Hatfield, PA, USA) and 0.15% picric acid (Sigma-Aldrich), and the resultant was blocked with Dulbecco's phosphate-buffered saline (DPBS) containing 3% bovine serum albumin (BSA; Thermo Fisher Scientific) and 0.3% Triton X-100 (Sigma-Aldrich) for 1 hour at room temperature, and then permeabilized. Subsequently, all samples were incubated overnight at 4°C with a primary antibody solution diluted in DPBS containing 1% BSA. The primary antibodies used are as described in Table 2 below.

**[Table 2]**

| **Antibody** | **Dilution** | **Supplier (Catalog Number)** |
|---|---|---|
| Rat anti-CORIN | 1:100 | R&D systems (#MAB2209) |
| Mouse anti-EN1 | 1:30 | DSHB (#4Gll.c) |
| Sheep anti-HOXB 1 | 1:500 | R&D systems (#AF6318) |
| Rabbit anti-PAX6 | 1:300 | BioLegend (#901302) |
| Rabbit anti-PAX2 | 1:100 | BioLegend (#901001) |
| Mouse anti-PAX5 | 1:50 | BD (#610862) |
| Mouse anti-KI67 | 1:500 | BD (#556003) |
| Mouse anti-TUJ1 | 1:2000 | BioLegend (#801213) |
| Rabbit anti-TUJ1 | 1:2000 | BioLegend (#802001) |
| Mouse anti-TH | 1:1000 | Sigma-Aldrich (#T1299) |
| Rabbit anti-TH | 1:1000 | Millipore (#AB152) |
| Mouse anti-FOXA2 | 1:200 | Abcam (#ab60721) |
| Rabbit anti-NURRl | 1:500 | Santa cruz (#sc-991) |
| Rabbit anti-LMXIA | 1:1000 | Millipore (#AB 10533) |
| Rabbit anti-TPH2 | 1:1000 | Novus Biologicals (#NB 100-74555) |
| Rabbit anti-vGLUTl | 1:1000 | Synaptic Systems (#135 303) |
| Rabbit anti-GABA | 1:1000 | Sigma-Aldrich(#A2052) |
| Goat anti-CHAT | 1:1000 | Millipore (#AB144P) |
| Rabbit anti-GFAP | 1:500 | Dako (#Z0334) |
| Mouse anti-04 | 1:50 | Millipore (#MAB345) |
| Rabbit anti-CALB | 1:2000 | Swant (#D-28k) |
| Chicken anti-MAP2 | 1:5000 | Abcam (#ab5392) |
| Mouse anti-NEUN | 1:50 | Millipore (#MAB377) |
| Rabbit anti-SYNAPSIN-I | 1:2000 | Millipore (#AB1543) |
| Goat anti-GIRK2 | 1:200 | Abcam (#ab65096) |
| Rabbit anti-VMAT2 | 1:200 | Millipore (#AB 1598P) |
| Rat anti-DAT | 1:500 | Millipore (#MAB369) |

Samples were washed 3 times with DPBS containing 0.1% BSA, and then incubated with Alexa Fluor 488- or Alexa Fluor 594-conjugated secondary antibodies (both Thermo Fisher Scientific) for 1 hour at room temperature. Nuclei were stained using Hoechst 33342 (Ho.; Thermo Fisher Scientific), and fluorescence images were obtained using a Leica DMI4000B microscope (Leica, Wetzlar, Germany) and Olympus FV1000 confocal microscope (Olympus, Tokyo, Japan).

### Example 4. Performance of chromatin immunoprecipitation-sequencing

To perform chromatin immunoprecipitation-sequencing (ChIP-seq), a SampleChIP enzyme chromatin IP kit (Cell Signaling Technology, Danvers, MA, USA) was used.

After cross-linking 4 million cells with 1% formaldehyde for 10 minutes at room temperature, 0.125 M glycine was added thereto and the cells were cultured for 10 minutes. Subsequently, the cells were washed with DPBS and continuously cultured with three cell lysates according to the manufacturer's instructions to damage cells so as to isolate chromatins.

The isolated chromatins were sonicated for 10 to 18 cycles (30 seconds on/30 seconds off) using Bioruptor Pico (Diagenode, Seraing, Belgium), and then incubated overnight at 4°C with histone and IgG control antibodies. After cross-linking the immunoprecipitated chromatins to Protein G magnetic beads, the resultant was subjected to a DNA purification process. The ChIP-seq library was constructed using a NEBNext ultra DNA library prep kit for Illumina platform (New England BioLabs, Ipswich, MA, USA) and sequencing was performing using the Illumina HiSeq 2000 (Illumina, San Diego, CA, USA).

The raw data of ChIP-seq was pre-processed with Bowtie2 (Version 2. 2. 6) and analyzed with MACS software (version 1. 4. 2) (Feng et al., 2011; Langmead and Salzberg, 2012).

In addition, in order to select genes with acquisition or loss of the H3K4me3 mark in the promoter region during direct reprogramming of hiDPs, standardized tags were analyzed and visualized in a heat map format using R.

GO analysis using DAVID (https://david.ncifcrf.gov/summary.jsp) was performed using the gene list of acquisition or loss of the H3K4me3 mark, and the upper GO biological process was visualized with the Microsoft Excel (Version 16. 16. 11).

Gene listings of "midbrain development" and "nervous system development" were obtained from QuickGO (https://www.ebi.ac.uk/QuickGO/), and the results of ChIP-seq on all listed genes were visualized by box plotting using R.

Integrative Genomics Viewer (IGV; Version 2. 3. 91) was used to analyze the ChIP-seq signal at a specific genomic location.

### Example 5. Microarray analysis

The overall gene expression profile was analyzed with the Agilent Human GE 4 X 44K (V2) chip (Agilent Technologies, Santa Clara, CA, USA). Briefly, RNA quality of all samples was checked with the Agilent 2100 Bioanalyzer System, followed by amplification, labeling, and hybridization steps. Raw data were standardized using the Agilent's GeneSpringGX software (Version 7. 3. 1).

Further analysis was performed after the numerical values of the raw data were converted to log2-conversion data or Z-score. The expression profile of the selected gene was visualized in a heat map format using the R (version 3. 2. 3, https://cran.r-project.org/bin/windows/base) package "gplot".

Principal Components Analysis (PCA) and Scatter plot were analyzed and visualized using R. Functionally classified Gene Ontology (GO) / signal path (pathway) was analyzed along with ClueGO plug-in (Version 2.2.5, http://apps.cytoscape.org/apps/cluego) using Cytoscape software platform (version 3.3.0, http://www.cytoscape.org/what_is_cytoscape.html). For comparison of the obtained data, published microarray data (GSE74991) was downloaded from NCBI GEO and used.

### Example 6. Electrophysiological analysis

Whole-cell patch-clamp recordings were performed to measure spontaneous or evoked action potentials (APs) and voltage-gated sodium/potassium currents. The cells plated on the coverslip were placed in a recording chamber and continuously perfused with a 35°C bath solution containing 137 mM NaCl, 2.0 mM CaCl₂, 10 mM HEPES, and 20 mM glucose (pH 7.3 adjusted with NaOH) (5 mL/min).

Patch pipettes were manufactured with borosilicate glass capillaries (Clark Electromedical Instruments, UK) and operated using a PP-830 pipette puller (Narishige Scientific Instrument Lab.; Tokyo, Japan). The resistance of the pipette was 5-6 MΩ when filled with a pipette solution containing 140 mM K-gluconate, 5 mM NaCl, 1 mM MgCl₂, 0.5 mM EGTA, and 10 mM HEPES (pH 7.25 adjusted with KOH).

Voltage- or current-clamp protocol generation and data acquisition were controlled by a computer, which was equipped with a Digidata 1440 A/D converter (Molecular Devices, San Jose, CA, USA) and pCLAMP 10.3 software (Molecular Devices). The signals were filtered at 5 kHz and sampled at 10 kHz using an Axopatch 200B amplifier (Molecular Devices).

Spontaneous APs firing were recorded in an I-clamp mode without current injection (I = 0), and rebound APs were induced with a short injection of hyperpolarized current (-20 pA, 0.2 s). In addition, the induced APs were recorded through a stepwise increase of the current by 0.02-nA from -0.1 nA to 0.2 nA for 1 second. In order to measure the voltage-switching current, the current was injected through a stepwise increase by 20-mV from -70 mV to +110 mV for 1 second.

### Example 7. Analysis of CGH array

In order to measure copy number alterations (CNAs) by direct reprogramming and long-term subculture, microarray of genome hybridization (Comparative Genomic Hybridization array; CGH array) was performed.

Genomic DNA was extracted from cells using the Wizard Genomic DNA Purification Kit (Promega, Madison, WI, USA), and DNA concentration and purity were measured using the ND-1000 spectrophotometer (Nanodrop Technologies, Wilmington, DE, USA). DNA quality was confirmed by electrophoresis on a 2% agarose gel with a reference DNA (Agilent Technologies) to see if DNA degradation occurred.

To measure the copy number change (CNAs), the SurePrint G3 Human CGH Microarray 4 Y 180 K kit (Agilent Technologies) was used. Agilent's male and female genomic DNA (normal individuals in Europe) were used as reference DNA. 1.5 µg of DNA was digested at 37°C for 2 hours using *Alu*I and *Rsa*I. Each of the cleaved samples obtained from fibroblasts and hiDPs (p7, p13, and p22) was labeled using Cy5-dUTP and Cy3-dUTP for reference DNA. The labeled DNA was separated using the SureTag DNA Labeling Kit Purification Columns (Agilent Technologies), and hybridization was performed with microarray slides at 65°C for 24 hours. Subsequently, the microarray slides were scanned and the raw data were extracted using the Agilent Feature Extraction software V10.7.3.1.

The raw data were analyzed using the Genomic Workbench 7.0.4.0 software (Agilent Technologies) under default settings with slight modifications. The threshold value was set to 6 in the ADM-2 algorithm, and probe mapping was performed according to genomic location within the UUCSC genome browser (Human NCBI37/hg19).

### Example 8. Confirmation of single nucleotide mutation and presence/absence of insertion/deletion

To confirm the presence/absence of gene mutations, single nucleotide variations (SNVs), and insertion/deletion (indel), genomic DNA was extracted from cells and NGS was performed using a customized panel which includes genes related to characteristics of tumors and stem cells.

The cultured cells were harvested using a scraper, lysed, and then genomic DNA was extracted using a DNeasy Blood & Tissue kit (Qiagen) according to the manufacturer's instructions.

The extracted genomic DNA was measured by quantitative fluoroscopic analysis using a Qubit 3.0 Fluorometer (Thermo Fisher Scientific) together with a Qubit DsDNA HS analysis kit (Qiagen). The final concentration of the input DNA was adjusted to 0.67 ng/µL. The library was prepared so as to conform to the Ion Chef System (Thermo Fisher Scientific) according to the manufacturer's instructions. Subsequently, the elaborated library was sequenced using the Ion 540 Chip (Thermo Fisher Scientific) and the Ion 540 kit-Chef Kit (Thermo Fisher Scientific).

The obtained sequencing raw data were adjusted to hg19 human reference genome using the Torrent Mapping Alignment Program aligner of the Torrent Suite software (Thermo Fisher Scientific, v5.10) in a FASTQ format. SNV calling to generate a variant call format file was performed using the Oncomine Variant Annotator v2.3 (Thermo Fisher Scientific) plug-in. The unfiltered files extracted from the TSV format were analyzed based on the following criteria: only SNVs and MNVs (Multi Nucleotide Variations) were selected as mutation types, and exons and splice sites were included except the introns and UTRs at the analysis site.

### Experimental Example 1. Characterization of hiDPs produced from human

### neonatal fibroblasts

### Experimental Example 1.1. Analysis of expression of midbrain-specific gene

### markers

For the hiDPs obtained in Example 1.2 and the hiNSCs obtained in Comparative Example 1, the presence/absence of expression and expression levels of midbrain-specific gene markers were compared.

CORIN is a specific marker for DPs and is used as a surface antigen to enrich only DPs, among the mesenchymal cells differentiated from pluripotent stem cells (PSCs). It was reported that the transplantation of enriched and isolated CORIN⁺ cells using an antibody against CORIN showed therapeutic effects in PD rodent and primate models.

The hiDPs obtained in Example 1.2 and the hiNSCs obtained in Comparative Example 1 were subjected to immunocytochemical staining for CORIN by the method described in Example 3. As a result, a positive signal was detected in hiDPs, but no positive signal was detected in hiNSCs derived from the same neuroectodermal lineage (FIG. 7).

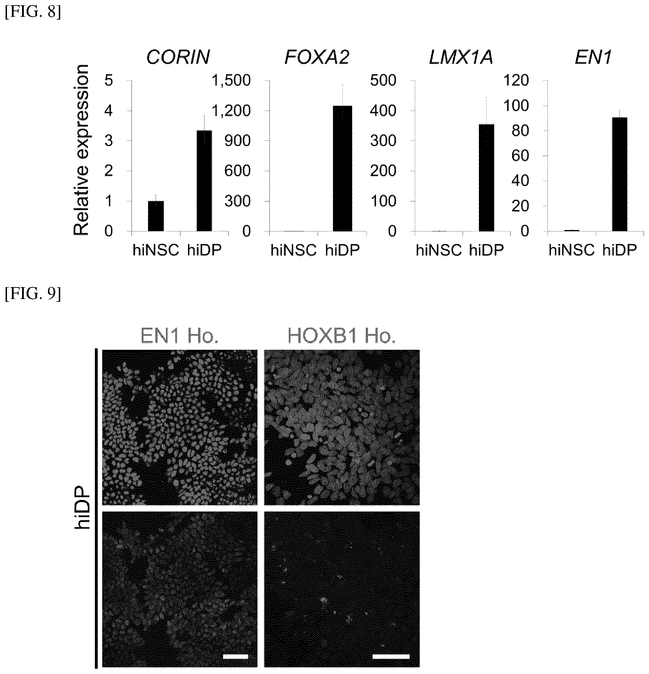

In addition, in order to compare the expression levels of the midbrain specific markers of hiDPs obtained in Example 1.2 and the hiNSCs obtained in Comparative Example 1, qRT-PCR was performed by the method described in Example 2. It was confirmed that not only CORIN, but also FOXA2, LMX1A, and EN1, which are known to be abundant in floor plate cells, exhibited higher expression levels in hiDPs than in hiNSCs (FIG. 8).

In order to confirm the regional identity of the hiDPs obtained in Example 1.2, immunocytochemical staining for midbrain (EN1) and hindbrain (HOXB1) specific markers were performed by the method described in Example 3. As a result, it was confirmed that EN1 was expressed in most hiDPs, but HOXB1 expression was not detected (FIG. 9).

From these results, it was confirmed that the hiDPs of the present disclosure have midbrain-specific properties.

### Experimental Example 1.2. Evaulation of proliferation potential

As previously known, since one of the major problems with the clinical application of PSC-derived DPs (PSC-DPs) is a gradual change in cellular properties (e.g., limited proliferation and specific marker expression), an experiment was performed to confirm whether the hiDPs of the present disclosure can fulfill proliferation throughout the successive subcultures.

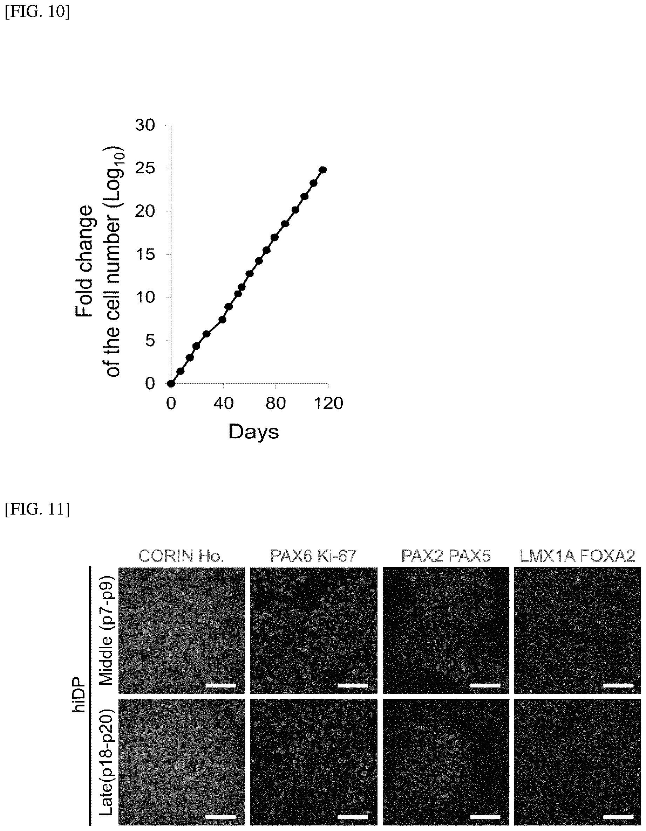

As a result of measuring the number of cells increased in the process of culturing the hiDPs obtained in Example 1.2 for 120 days or more, it was found that the proliferation rate was constant and the number of cells increased by 10²⁴ times or higher, thus confirming the proliferation potential (FIG. 10 ).

In order to evaluate whether or not the characteristics of hiDPs themselves are maintained during long-term culture, the hiDPs in intermediate (7-9) and late (18-20) subcultures were analyzed.

In the intermediate and late subcultures of the hiDPs obtained in Example 1.2, immunocytochemical staining were performed for Ki-67 (a cell cycle marker) and CORIN, PAX2, PAX5, and FOXA2 (DPs-specific markers) by the method described in Example 3. As a result, it was confirmed that the markers were constantly expressed (FIG. 11). In particular, PAX2 and PAX5 are known to be expressed at the midbrain-hindbrain boundary, which is closely correlated with the ability to differentiate into dopaminergic neurons after implantation *in vivo.* Meanwhile, as described in Experimental Example 1.1, LMX1A, in which expression was confirmed at the mRNA level, could not be confirmed regarding its expression at the protein level (FIGS. 8 and 11).

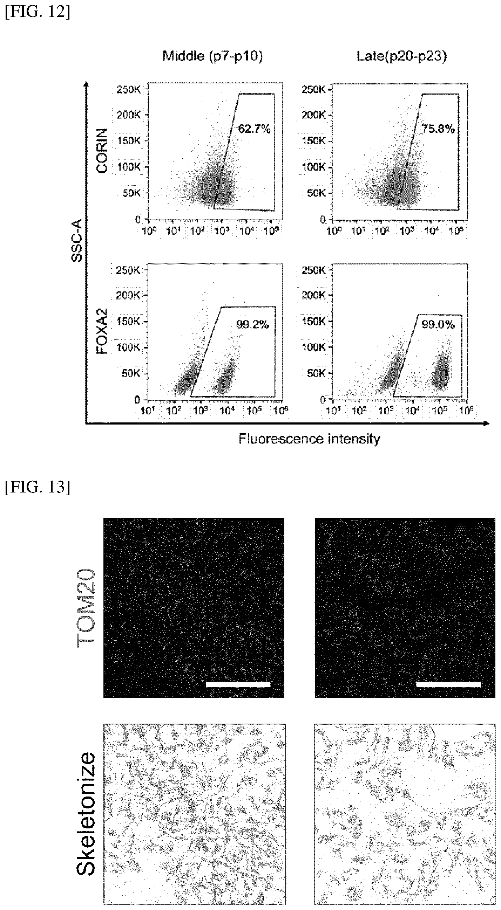

In addition, as a result of performing quantitative analysis on CORIN and FOXA2 through flow cytometry, it was confirmed that the hiDPs obtained in Example 1.2 were positive by 62% or more and 99% or more for CORIN and FOXA2, respectively, even after 7 or more times of subcultures and 20 or more times of subcultures (FIG. 12).

From these results, it was confirmed that the hiDPs of the present disclosure enable proliferation while maintaining highly pure midbrain-specific characteristics even through multiple subcultures.

### Experimental Example 1.3. Analysis of mitochondrial structure

It is known that highly proliferative cells (*e.g.*, PSCs, neural stem cells, hematopoietic stem cells, mesenchymal stem cells, and cancer cells) have metabolic reactions that are highly dependent on glycolysis suitable for producing components for the synthesis of cellular energy requirements and other essential products. Moreover, glycolysis is closely associated with an immature mitochondrial structure. Therefore, the mitochondrial structures of hiDPs obtained in Example 1.2 and the PSC-DPs obtained in Comparative Example 3 as a control were analyzed.

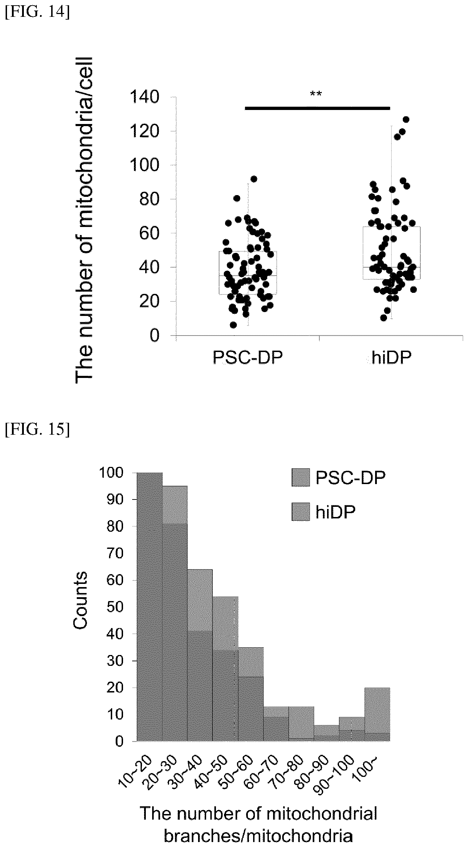

To compare the mitochondrial morphology, immunocytochemical staining was performed for TOM20 (which is a mitochondrial-specific marker) using the hiDPs obtained in Example 1.2 and the PSC-DPs obtained in Comparative Example 3 by the method described in Example 3. For analysis, the original fluorescence image for TOM20 was skeletonized. As a result, it was confirmed that there were a greater number of mitochondria per cell in the hiDPs compared to the PSC-DPs (FIGS. 13 and 14).

In addition, as a result of analyzing the number of branches per mitochondria, it was confirmed that hiDPs had a simpler mitochondrial structure (less than 40 branches) compared to PSC-DPs (FIG. 15).

From these results, it was confirmed that the hiDPs of the present disclosure have immature mitochondria for glycolysis compared to PSC-DPs, and this characteristic is related to the very high proliferation property of hiDPs.

**Experimental Example 2. Analysis of changes in cell characteristics during**

### direct reprogramming of hiDPs

### Experimental Example 2.1. Analysis of changes in genetic characteristics

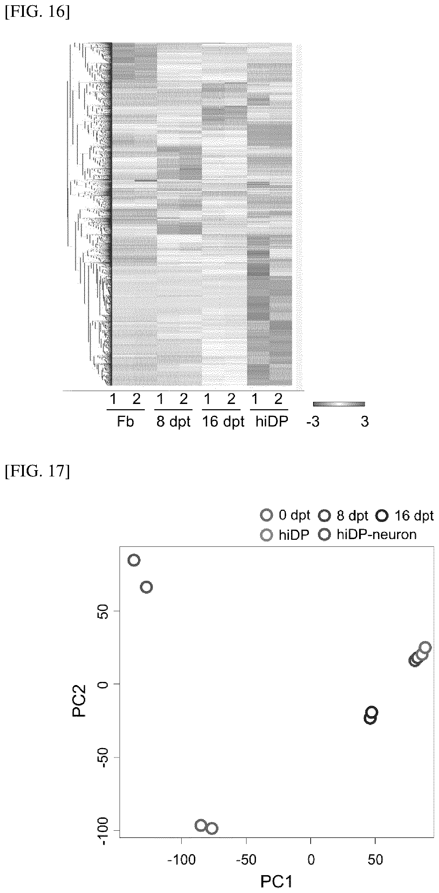

In order to compare the transcriptomes of the hiDPs obtained from parental fibroblasts (CRL-2097; Fb), the direct reprogramming process (8 dpt and 16 dpt), and in Example 1.2, microarrays were performed by the method described in Example 5. It was confirmed that the overall gene expression was changed during the direct reprogramming of hiDPs by differentially expressed gene (DEG) analysis and PCA (FIGS. 16 and 17).

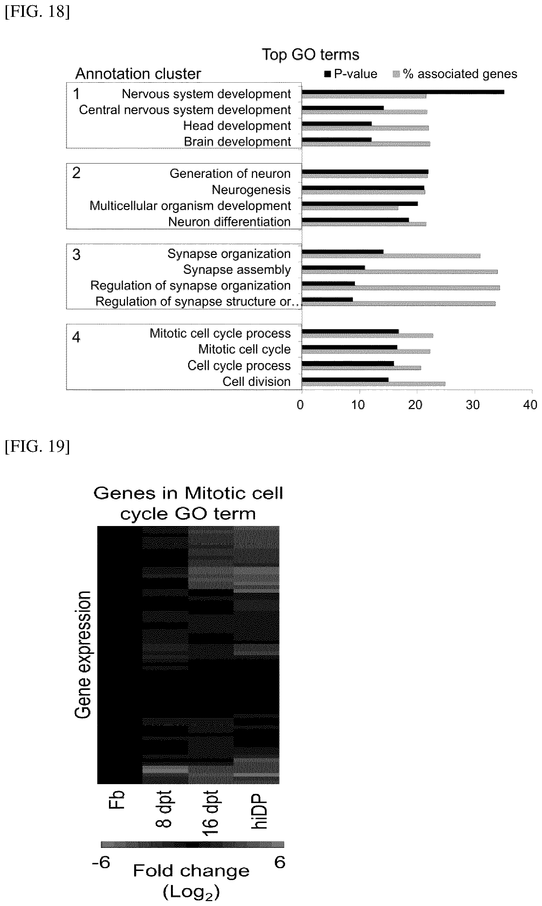

In order to obtain information on the function of DEG, the GO terms enrichment analysis was performed by DAVID function annotation clustering. As a result, it was confirmed that the genes significantly upregulated (a change of a 5-fold or higher) in hiDPs were related to the development of the nervous system, the generation of neurons, synaptic organization, and mitotic cell cycle processes (consistent with the long-term proliferative potential) (FIG. 18).

In order to evaluate the expression levels of the genes included in the GO terms of the mitotic cell cycle process, a heat map analysis of the genes was performed, and it was confirmed that as the direct reprogramming from fibroblasts to hiDPs proceeded, the overall increase in the expression of related genes was observed (FIG. 19).

From these results, it was confirmed that hiDPs acquired the genetic characteristics of the neural system cells, particularly the central nervous system, capable of proliferation during the direct reprogramming process.

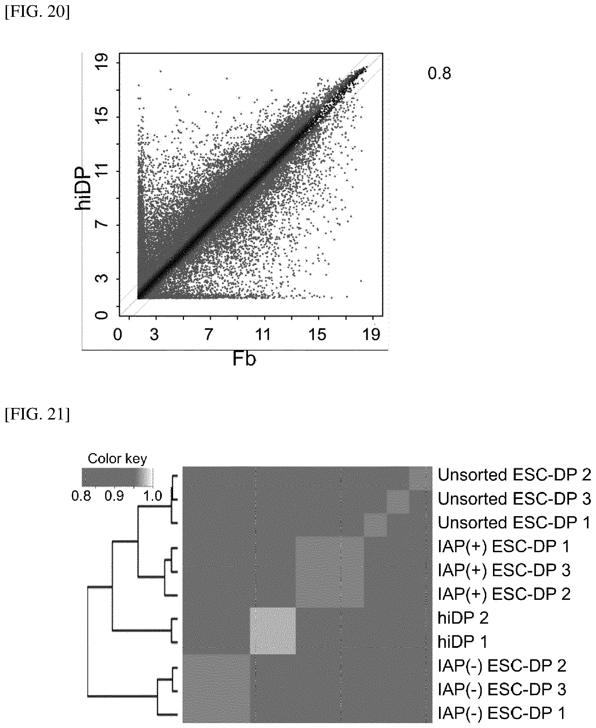

In addition, as a result of comparing the transcriptome profiles of parental fibroblasts (Fb) and hiDPs produced therefrom through scattering analysis, a distinct difference was shown (FIG. 20).

In order to compare the properties of the hiDPs obtained in Example 1.2 and the DPs previously reported to be implantable, hiDPs transcriptome data and publicly available embryonic stem cell (ESP)-derived DP data; that is, a cluster analysis between integrated associated protein (IAP)-positive classified, IAP-negative classified, and unclassified data was performed. IAP is a reliable marker of dopaminergic cells present in the midbrain, and IAP-positive classified cells showed an improved functional recovery compared to unclassified cells in the PD rat model. As a result of the analysis, it was confirmed that the hiDPs obtained in Example 1.2 were more similar to the IAP-positive classified DPs compared to the IAP-negative classified DPs and the unclassified DPs (FIG. 21).

From these results, the potential of the hiDPs of the present disclosure in regenerative medicine for PD patients was confirmed.

### Experimental Example 2.2. Analysis of changes in epigenetic characteristics

Since the change in cell fate involves an epigenetic mechanism, it was assumed that the direct reprogramming condition for hiDPs of Example 1 may promote the epigenetic change into an open chromatin structure capable of accepting neurogenic signals. To prove this, genome-wide epigenetic changes were analyzed through ChIP-seq analysis for active (H3K4me3 and H3K27ac) chromatin marks of the hiDPs obtained from parental fibroblasts (Fb), 16 dpt and Example 1.2.

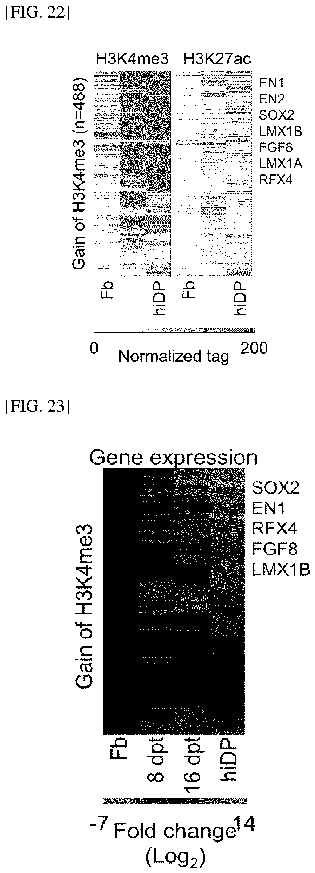

As a result of ChIP-seq analysis, it was confirmed that H3K4me3 significantly increased near the transcription start site (TSS) of multiple genes during the direct reprogramming process. In addition, these regions showed a tendency to simultaneously accumulate H3K27ac, and included promoter regions of midbrain marker genes (*e.g*., EN1, EN2, SOX2, LMX1B, FGF8, LMX1A, and RFX4) (FIG. 22). In addition, through heat map analysis, it was confirmed that the expression of genes related to these regions was upregulated during the direct reprogramming of hiDPs (FIG. 23).

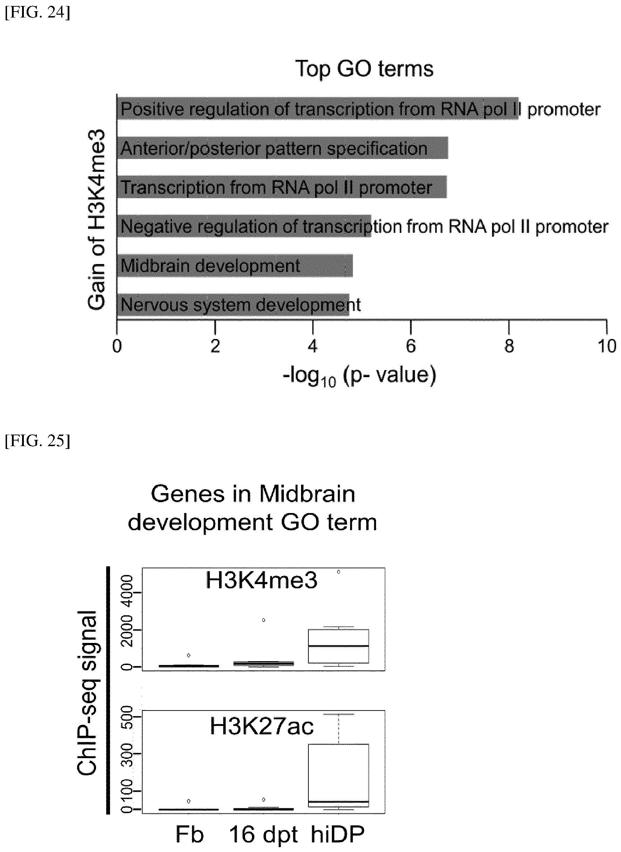

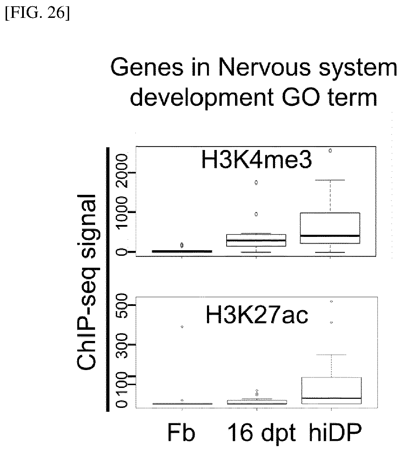

GO-term analysis indicated that these genomic locations were particularly related to "transcriptional regulation", "nerve development", and "midbrain development" GO-terms, which means that the induction of overall neuronal gene expression (FIG. 24). In addition, the increase in H3K4me3 and H3K27ac was detected in the promoter regions of the genes related to "midbrain development" and "nervous system development" (FIGS. 25 and 26).

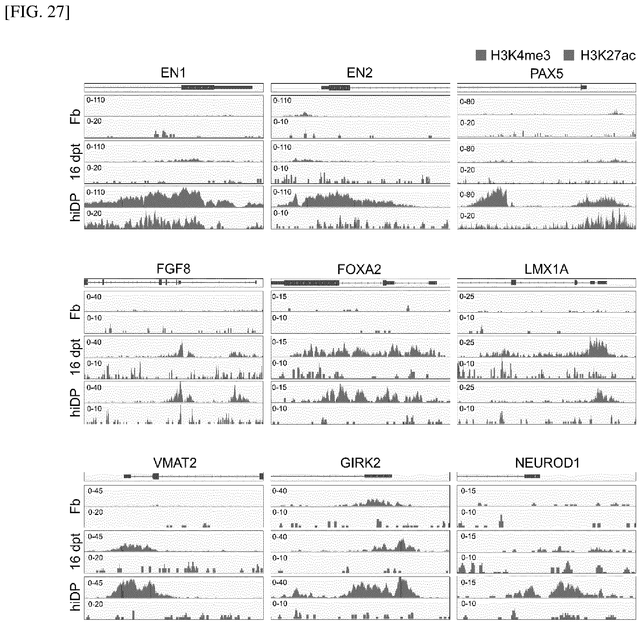

Specifically, as a result of analyzing the changes in the H3K4me3 and H3K27ac histone marks, it was confirmed that the promoter regions of the midbrain-hindbrain boundary-specific genes (EN1, EN2, and PAX5) and midbrain basal plate-specific genes (FGF8, FOXA2, and LMX1A) acquired H3K4me3 and H3K27ac during the direct reprogramming of hiDPs, whereas the promoters of the dopaminergic neuronal genes (VAMT2, GIRK2, and NEUROD1) showed a chromatin structure that was already open before differentiation (FIG. 27).

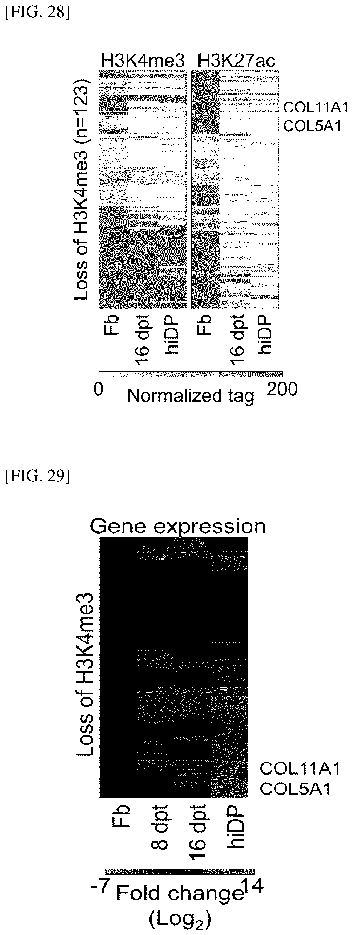

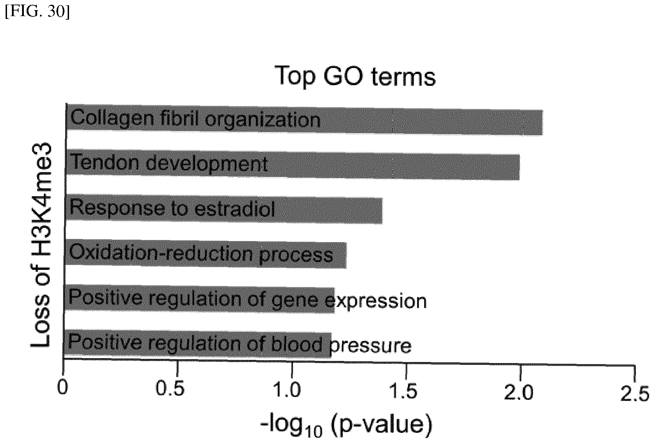

It was confirmed that there are also many genes in which H3K4me3 gradually disappears in the genome near the TSS. In these genes, H3K27ac was gradually disappeared, and hiDPs were downregulated during direct reprogramming (FIGS. 28 and 29). It was confirmed that these genes are related to the gene set of collagen fibril organization which is highly expressed in fibroblasts (FIG. 30).

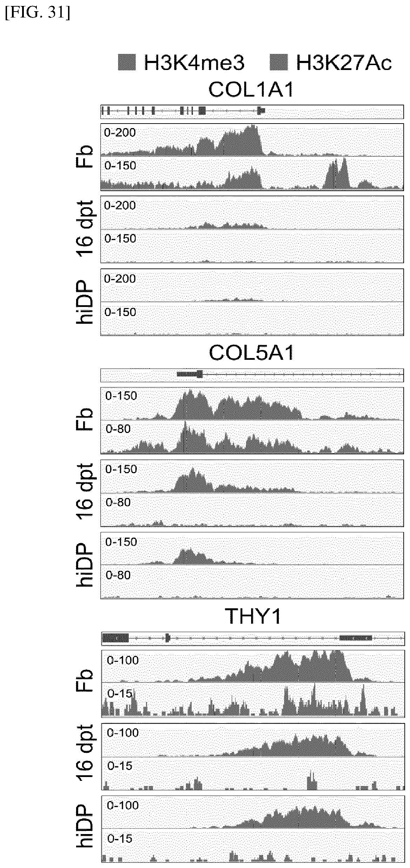

Specifically, the genomic locations of COL1A1, COL5A1, and THY1 gradually lost their active chromatin marks (FIG. 31).

From these results, it was confirmed that the conversion of the chromatin state facilitates the accessibility of the midbrain development signal to the gene location related to midbrain development, thereby enabling direct reprogramming of hiDPs.

### Experimental Example 3. Differentiation of midbrain DNs from hiDPs

### Experimental Example 3.1. Confirmation of possibility of differentiation into midbrain DNs

In order to evaluate the possibility of differentiation into dopaminergic neurons (DNs), the hiDPs obtained in Example 1.2 and the hiNSCs obtained in Comparative Example 1 were cultured in a neuronal differentiation medium. Specifically, the hiDPs and the hiNSCs were plated at a density of 30,000 cells/mm² on Geltrex-coated plastic coverslips (diameter: 13 mm, Thermo Fisher Scientific). On the next day, the culture medium was replaced with a DMEM/F-12 based neuronal differentiation (ND) medium, which contained IX B27 (minus vitamin A), IX penicillin/streptomycin (Thermo Fisher Scientific), 20 ng/mL brain-derived neurotrophic factor (Peprotech), 20 ng/mL glial cell-derived neurotrophic factor (Peprotech), 0.5 mM dbcAMP (Enzo-Life Sciences, Basel, Switzerland), and 50 µg/mL 2-phospho-L-ascorbic acid. To initiate differentiation, 0.2 mM sodium butyrate and 0.1 µM Compound-E (a γ-secretase inhibitor; Millipore) were added for 1 week. The culture medium was replaced by half of the total amount every 3 days.

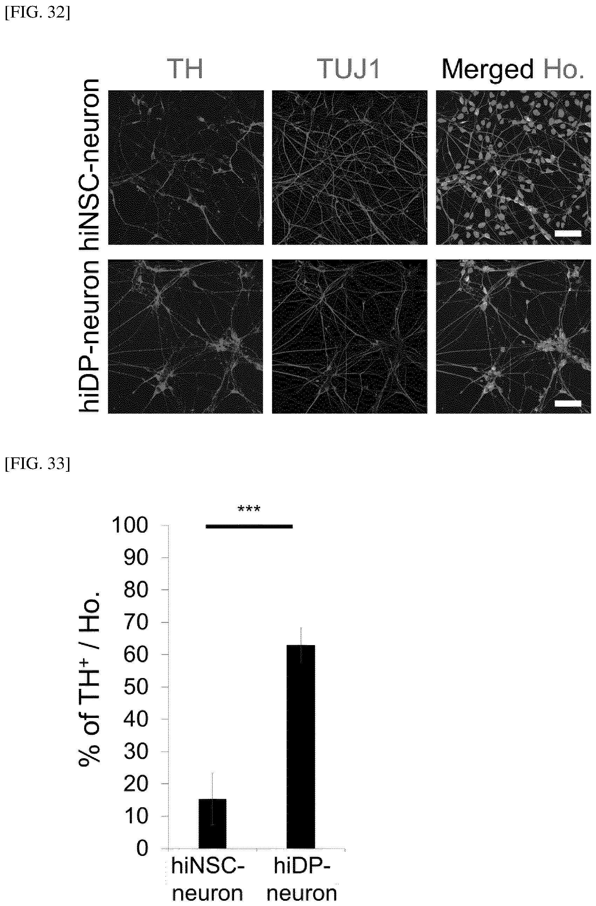

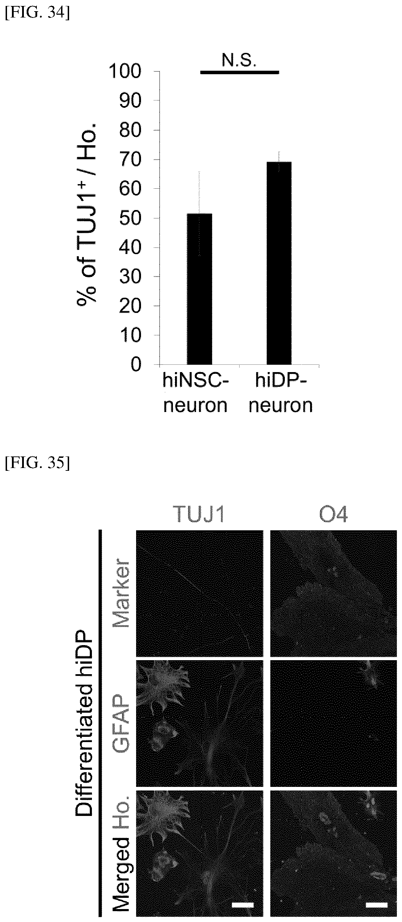

21 days after the initiation of differentiation, the hiDPs (62.9 ± 5.3%) produced a higher proportion of TH⁺ dopaminergic neurons than hiNSCs (15.3 ± 8.0%) (FIGS. 32 and 33). However, as a result of quantitative analysis after immunocytochemical staining with TUJ1 representing all neurons between these two types of cells, there was no significant difference in the amount of TUJ1⁺ neurons (FIG. 34).

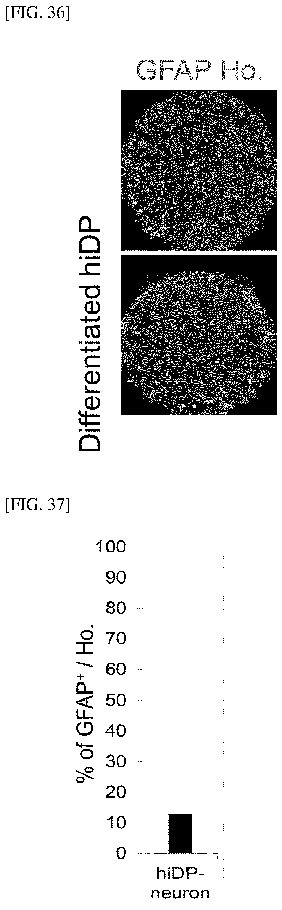

When the hiDPs obtained in Example 1.2 were cultured under neuronal differentiation conditions for 10 weeks or longer, it was confirmed that GFAP⁺ astrocytes (astroglia cells) were produced (FIG. 35). In addition, it was confirmed that a very small number of O4⁺ oligodendrocytes (about 2 to 4 cells among the total cells) also appeared (FIG. 35). In order to quantify the ratio of these glial cells, immunocytochemical staining was performed using the method described in Example 3, and then fluorescence image scanning was performed on the entire area (FIG. 36). As a result, it was confirmed that 11.7 ± 0.7% of the cells, which were differentiated from the hiDPs obtained in Example 1.2, were GFAP + astrocytes (FIG. 37).

From these results, it was confirmed that the hiDPs of the present disclosure were efficiently differentiated into midbrain-specific DNs and glial cells.

### Experimental Example 3.2. Characterization of differentiated midbrain DNs

### Experimental Example 3.2.1. Genetic characterization

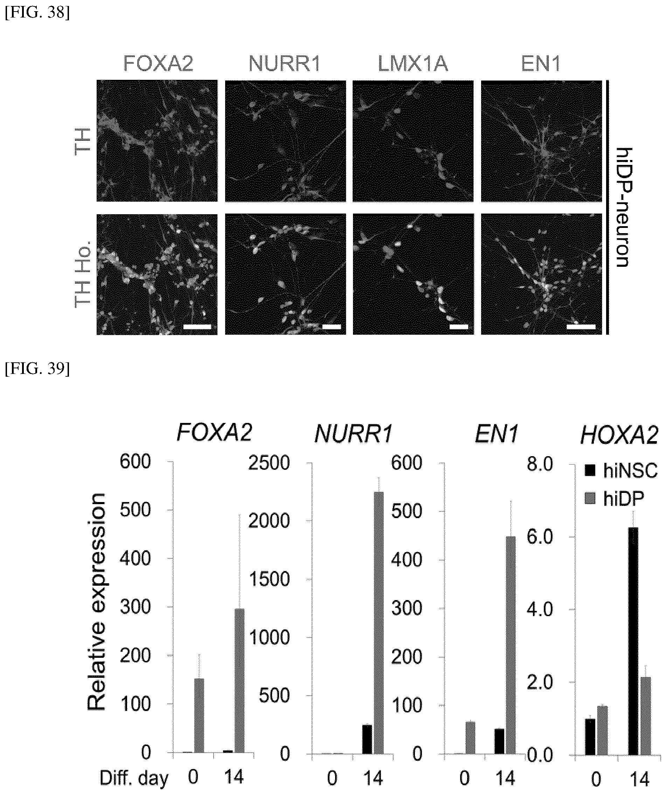

In order to characterize DNs differentiated from the hiDPs obtained in Example 1.2, immunocytochemical staining was performed for key markers of midbrain DNs by the method described in Example 3. As a result, most of the TH⁺ DNs co-expressed the midbrain markers FOXA2, NURR1, LMX1A, and EN1 (FIG. 38). In the intermediate and late subcultures of the hiDPs, as a result of performing immunocytochemical staining for LMX1A, the expression of LMX1A was not confirmed (FIG. 11), but the expression of LMX1A was confirmed in the DNs differentiated from hiDPs.

It was confirmed that the hiDPs had a relatively high level of expression of midbrain markers (FOXA2, NURR1, and EN1) after differentiation compared to before differentiation, and HOXA2 (a marker for hindbrain) was similarly expressed regardless of differentiation (FIG. 39).

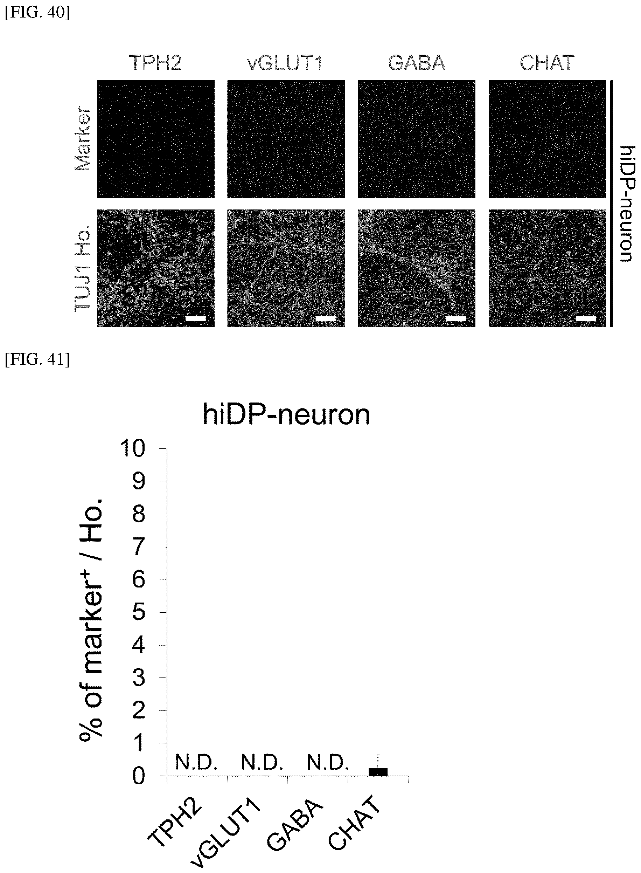

To investigate the proportion of different neuronal subtypes of the DNs differentiated from hiDPs, immunocytochemical staining was performed for specific markers against serotonergic (TPH2), glutamatergic (vGLUT1), GABAergic (GABA), and cholinergic (CHAT) neurons. As a result, some CHAT⁺ neurons were detected, while vGLUT1⁺, GABA⁺, or TPH2⁺ neurons were not detected (FIGS. 40 and 41).

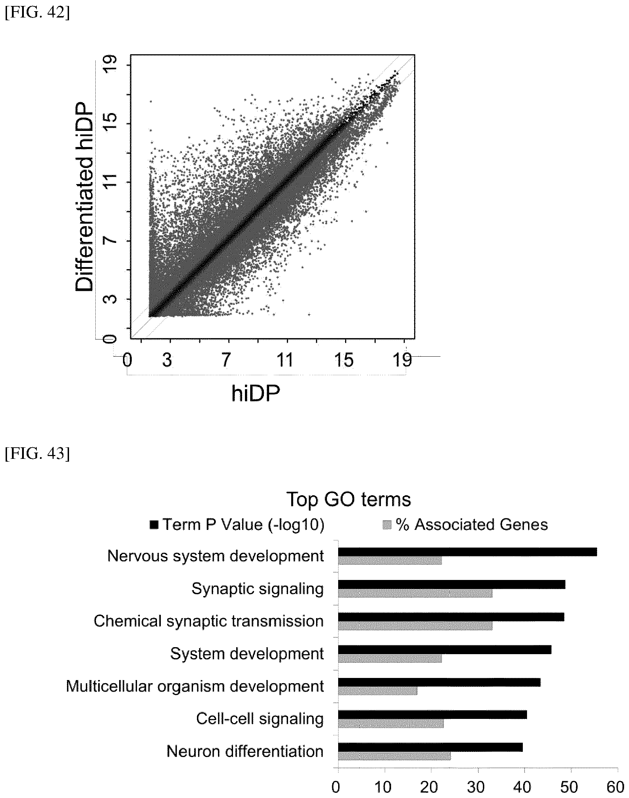

In addition, as a result of comparing the transcriptome profiles of the hiDPs and hiDPs-derived neurons through scattering analysis, distinct differences in gene expression patterns were shown (FIG. 42).

Through DAVID functional annotation clustering analysis, it was confirmed that the genes which were most remarkably upregulated (a 5-fold or more change) in the hiDPs-derived neurons were GO terms associated with "nerve development" and maturation (*e.g*., "synaptic signaling", "chemical synaptic transmission", and "neuron differentiation") (FIG. 43).

Considering the ChIP-seq data of the hiDPs before differentiation into DNs (FIG. 27), it is predicted that rapid changes in expression of genes associated with neuronal differentiation and maturation are possible because changes in the epigenetic state were already made before differentiation.

From these results, it was confirmed that the hiDPs of the present disclosure have a differentiation potential of highly pure midbrain-specific DNs, and this possibility was determined before differentiation.

### Experimental Example 3.2.2. Analysis of functional properties in vitro

In order to evaluate the *in vitro* functional properties of midbrain DNs differentiated from the hiDPs obtained in Example 1.2, immunocytochemical staining was performed on mature neuronal markers by the method described in Example 3.

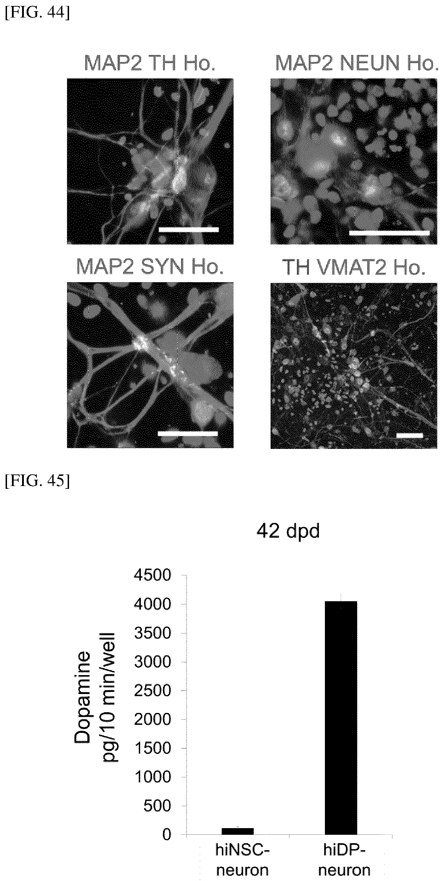

Most of the TH⁺ neurons were co-stained with MAP2 (a mature neuron marker), and MAP2⁺ neurons were co-stained with a neuron nucleus (NEUN) (another mature neuronal marker) (FIG. 44). In addition, MAP2⁺ neurons expressed SYNAPSIN-I (SYN), which is a presynaptic marker (FIG. 44).

In addition, quantitative analysis was performed to compare the levels of secreted dopamine between hiDPs-derived neurons and hiNSCs-derived neurons differentiated for 42 days. Specifically, the cells differentiated for 42 days were treated with 56 mM KCl (Sigma-Aldrich) for 10 minutes. Then, the entire culture medium was obtained and enzyme-linked immunoassay was performed using the Dopamine Research ELISA kit (LDN, Nordhorn, Germany). The absorbance value of the sample was compared with a standard curve prepared using a sample of a standard concentration. As a result, the neurons differentiated from hiDPs secreted a much higher level of dopamine than the neurons differentiated from hiNSCs (FIG. 45).

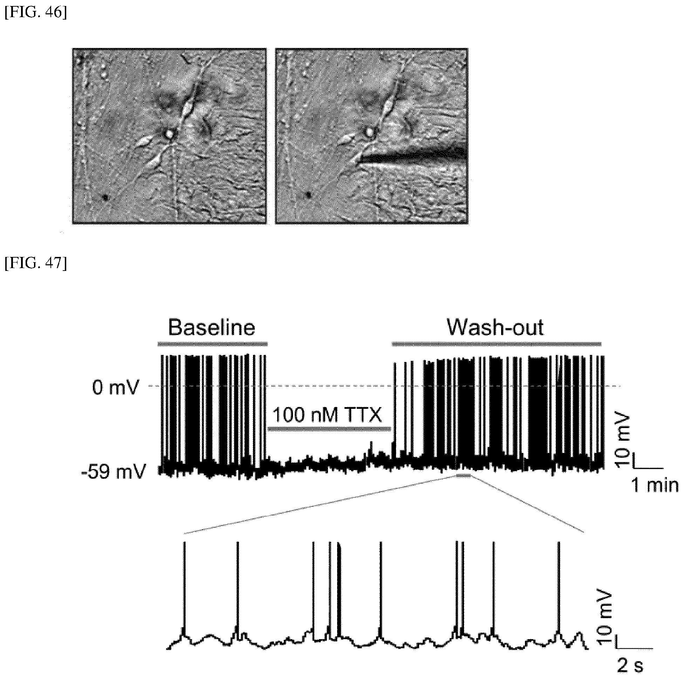

In order to evaluate whether hiDPs-derived neurons have functional membrane properties similar to those of the original neurons, whole-cell patch-clamp recording was performed by the method described in Example 6 on days 100 to 120 after the initiation of differentiation (FIG. 46). As a result, spontaneous APs were detected in 20 cells among the total cells (50%, n = 40; FIG. 47).

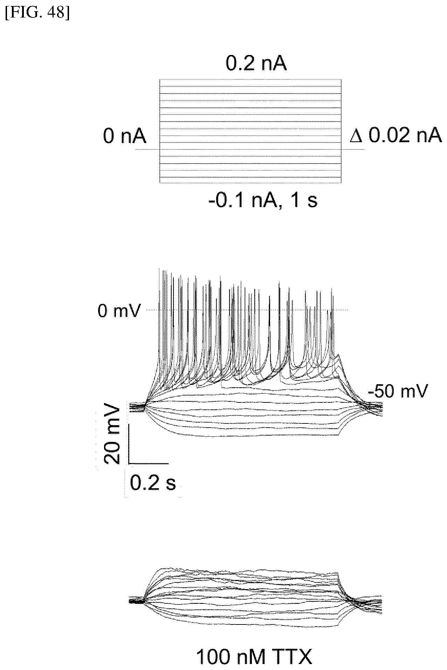

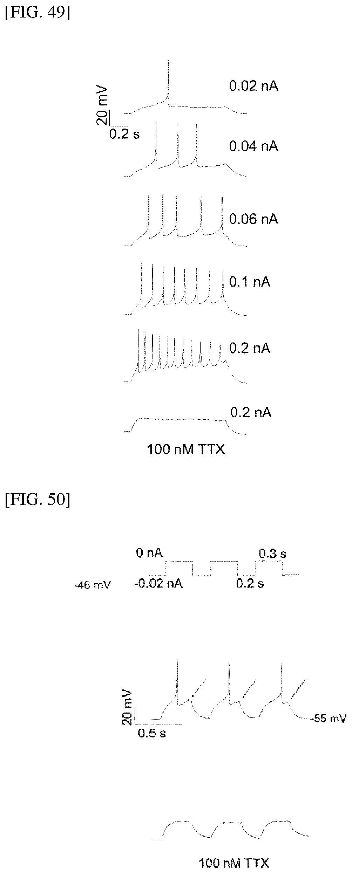

APs were also induced in most of the cells after injection of the depolarization current (FIG. 48), which was blocked by tetrodotoxin (TTX), a Na⁺ ion channel-specific inhibitor (FIG. 48), and AP firing was increased by the increase of the amount of the injection current (FIG. 49). In addition, rebound depolarization was induced by membrane hyperpolarization (FIG. 50). Spontaneous and induced APs are characteristics exhibiting the functionality of neurons, and rebound APs are known to be a characteristic of midbrain dopaminergic neurons.

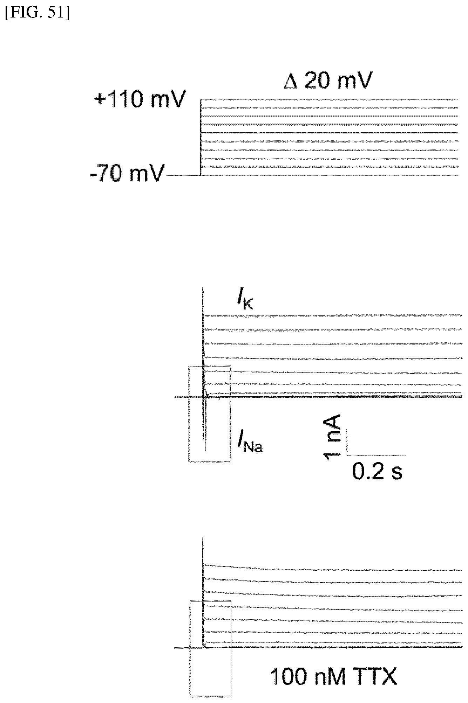

In a voltage-clamp mode, fast deactivation of both inner and outer currents was observed, which correspond to voltage-dependent K⁺- and Na⁺-channel currents. The inner Na⁺ channel current was completely blocked by TTX (FIG. 51). The resting membrane potential ranged from -43.6 mV to -68.4 mV, with an average of approximately -56.9 mV (n = 20).

From these results, it was confirmed that the neurons differentiated from the hiDPs of the present disclosure have functionality with regard to the ability to secrete dopamine and membrane properties *in vitro.*

### Experimental Example 4. Transplantation of hiDPs into PD mouse model

### Experimental Example 4.1. Evaluation of suitability of transplantation of hiDPs

Recently, some markers which can predict the therapeutic effect of PD after cell transplantation in cells which have not been transplanted were reported (Kirkeby *et al*., 2017). It was found that the well-known DPs-specific markers (FOXA2, LMX1A, and CORIN) had a negative correlation with the expression of predictive markers (EN1, PAX2, PAX5, PAX8, and SPRY1) for the outcome of transplantation.

Before cell transplantation to a PD animal model, in order to evaluate the transplantation suitability of the hiDPs obtained in Example 1.2 and the A-hiDPs obtained in Example 1.3, the expression levels of DPs-specific markers and therapeutic effect predictive markers (which are well known in the transcriptome profile) were compared by performing microarrays using the method described in Example 5.

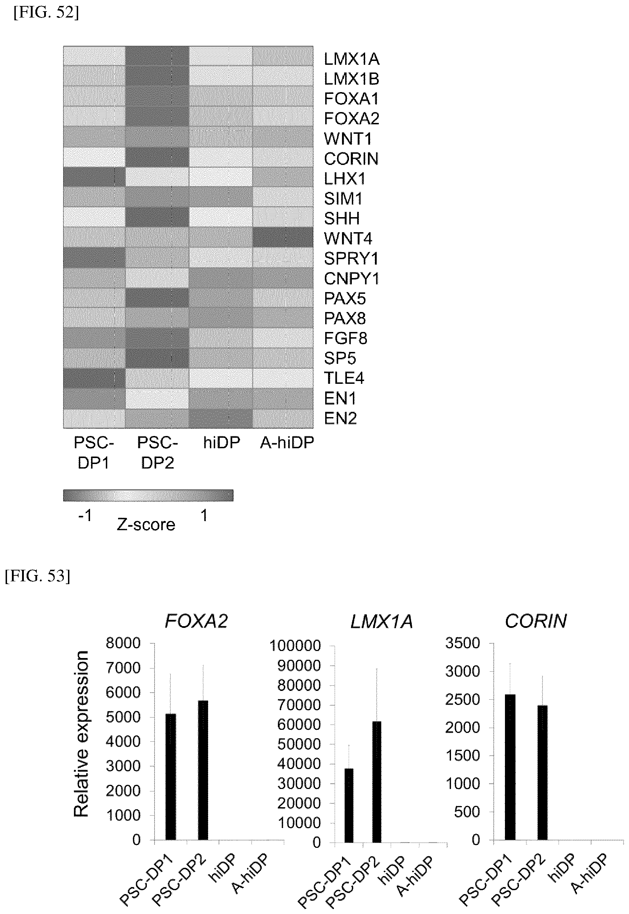

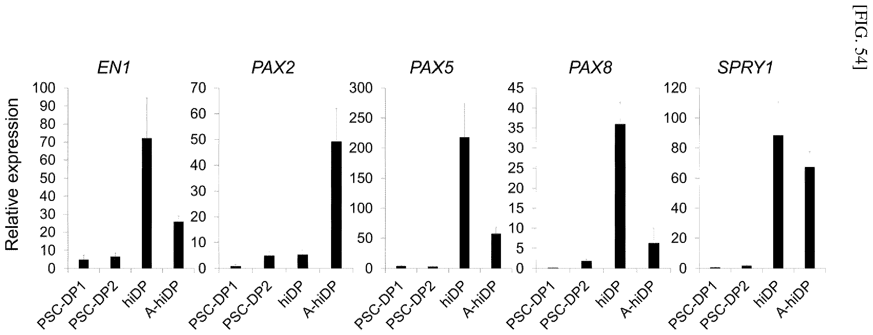

As a result, it was confirmed that the expression level of well-known DPs markers in the hiDPs obtained in Example 1.2 was low compared with PSC-DPs, whereas the level of predictive markers was higher (FIG. 52). These results were reconfirmed by qRT-PCR (FIGS. 53 and 54). Specifically, as a result of comparing the relative expression levels of well-known DPs-specific markers (FOXA2, LMX1A, and CORIN) based on the expression level of hiDP, it was confirmed that the expression level of PSC-DP was 1,000- to 100,000-fold higher than that of the hiDP. In contrast, as a result of comparing the relative expression levels of predictive markers (EN1, PAX2, PAX5, PAX8, and SPRY1) for the transplantation result based on the expression level of PSC-DP1, it was confirmed that the expression level of hiDP was 3- to 300-fold higher than that of PSC-DP.

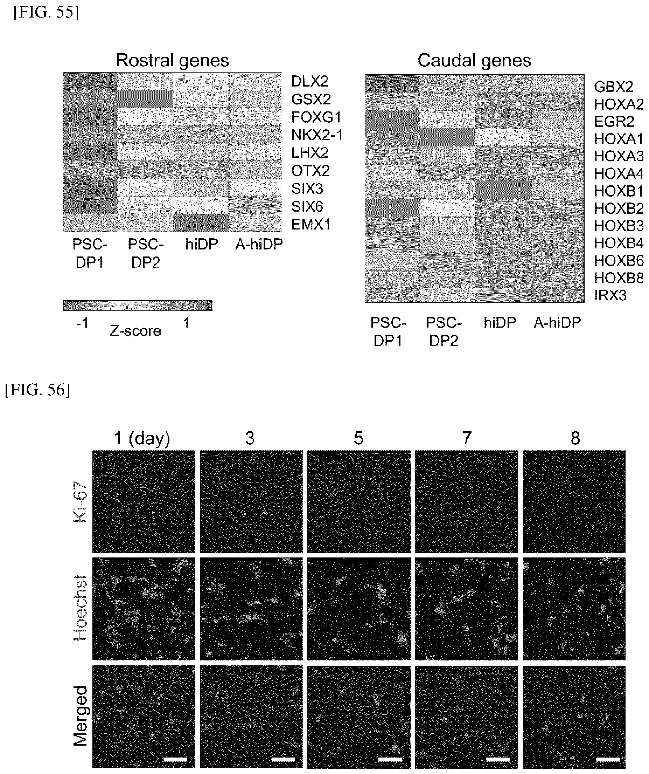

In addition, since DPs having the expression of a predictive marker are being reported to have the characteristic of the midbrain-hindbrain boundary, the expression levels of specific region markers in hiDPs and PSC-DPs were analyzed by performing microarrays using the method described in Example 5. The hiDPs showed a higher expression level of the caudal gene than the rostral gene, indicating tail midbrain identity (FIG. 55).

From these results, it was confirmed that the hiDPs of the present disclosure are separate cells which are distinguishable from PSC-DPs, and are more suitable for PD treatment through *in vivo* transplantation, compared to PSC-DPs.

### Experimental Example 4.2. Identifying suitalbe time for transplantation of hiDPs

In order to avoid tumor formation *in vivo* due to the nature of highly proliferative hiDPs, it was assumed to be appropriate to perform the transplantation of hiDPs when cell division no longer occurs.

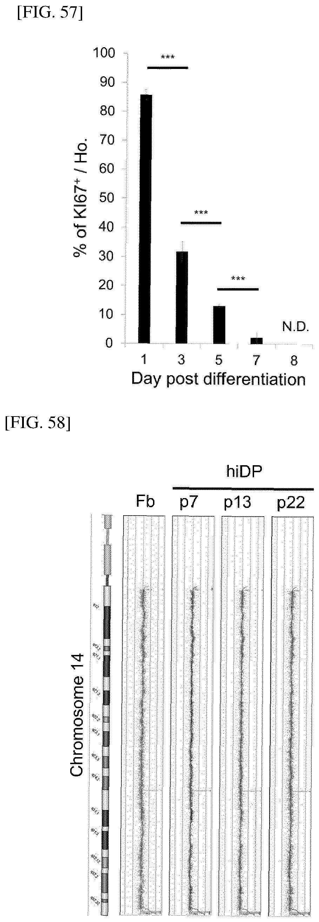

In order to confirm the time point at which cell division did not occur, immunocytochemical staining was performed for Ki-67 by the method described in Example 3, and the resultant was quantitatively analyzed. As a result, Ki-67⁺ cells exhibiting proliferative properties gradually decreased according to the differentiation conditions into neurons, and were not found on day 8 after differentiation (FIGS. 56 and 57). Therefore, it was determined to perform the transplantation of hiDPs on day 7 days after the differentiation into a PD mouse model.

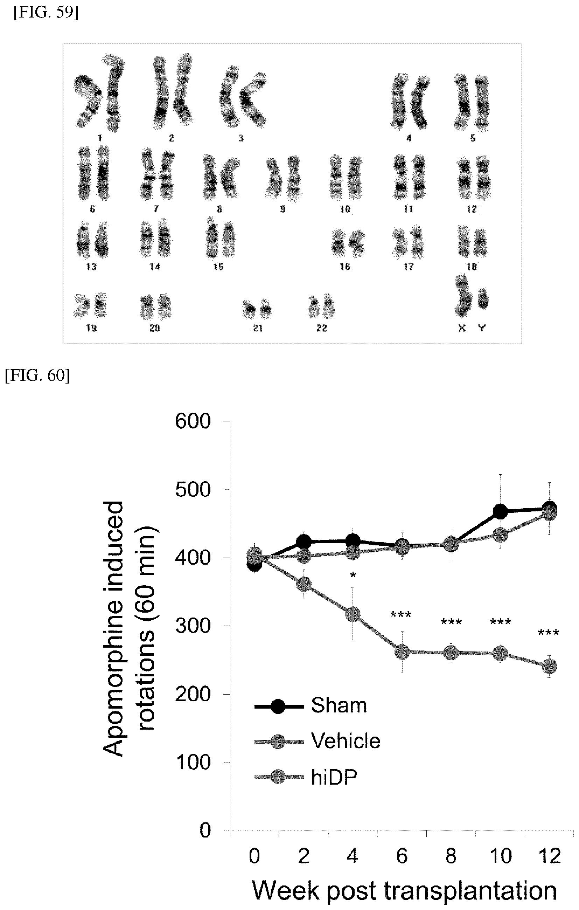

Meanwhile, in order to confirm whether the stability of the genome can be maintained even after a long-term subculture, a CGH array was performed by the method described in Example 7 to compare and analyze the changes in the copy number of hiDPs and their parental fibroblasts cultured for a long period of time. Compared to the parental fibroblasts, no CNVs were found in the hiDPs cultured for 22 subcultures (FIG. 58). In addition, it was confirmed through the karyotype analysis result that there was no abnormality in the chromosome structure of the hiDPs subcultured 24 times (FIG. 59).

From these results, it was confirmed that the genomic structure of hiDPs remained stable even after repeated divisions over a long period of time thus confirming that hiDPs are suitable for transplantation *in vivo.*

### Experimental Example 4.3. Confirmation of effect of hiDP transplantation on PD treatment

Normal mice were injected with 6-hydroxydopamine (6-OHDA; Sigma-Aldrich) into *substania nigra* of the midbrain under anesthesia with Avertin to create unilateral lesions (AP: -3.1 mm; ML: ±1.1 mm; DV: -4.4 mm). Apomorphine (0.4 mg/kg; Sigma-Aldrich) was injected before the formation of 6-OHDA-induced lesion and at 4 and 6 weeks after the formation so as to induce a rotational behavior, and this was measured to evaluate whether a Parkinson's disease model was created. Animals exhibiting 400 ± 50 rotations toward the lesion 60 minutes after the administration of apomorphine were determined to have created a Parkinson's disease model, and were used for transplantation of hiDPs.

A total of 1 × 10⁵ differentiated hiDP cells were transplanted into the lesion striatum of 6-OHDA-treated mice (AP: +0.4 mm; ML: ±1.5 mm; DV: -2.8 mm). Cyclosporin A was injected intraperitoneally daily for 7 days after the cell transplantation. The apomorphine-induced rotational behavior was measured every 2 weeks after the transplantation. The number of clockwise and counter-clockwise rotations were counted and expressed as total rotations per 60 minutes for the brain hemisphere. In order to minimize the bias, the behavioral analysis of the PD mouse model was independently evaluated by two experimenters in a blind manner.

In the 6-OHDA-induced PD mouse model, after the transplantation of hiDPs differentiated into neurons for 10 or more days, behavioral defects gradually recovered, whereas recovery was not observed in sham and vehicle control groups (FIG. 60). In addition, the hiDPs-injected mice showed a remarkable improvement in motor defects from the 4th week after the transplantation (FIG. 60).

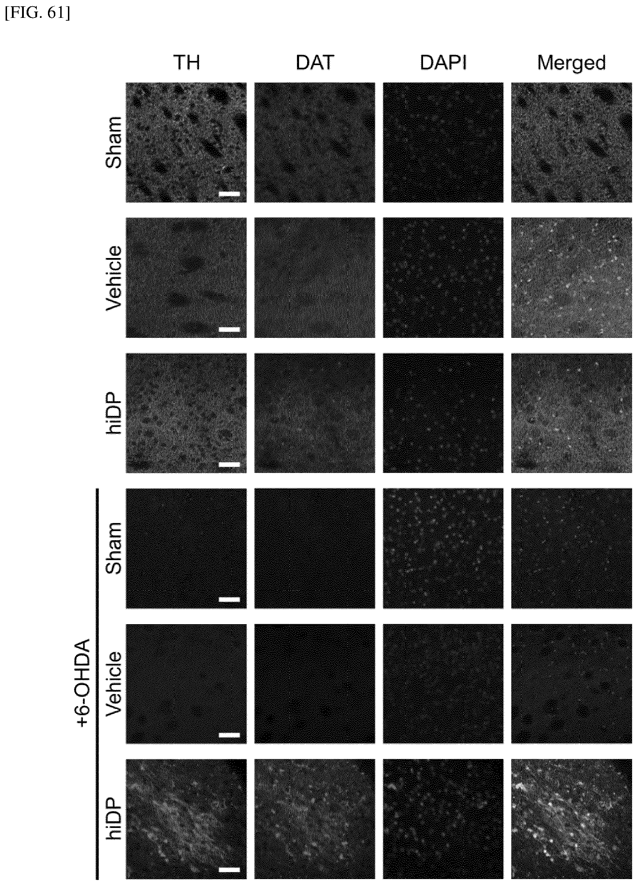

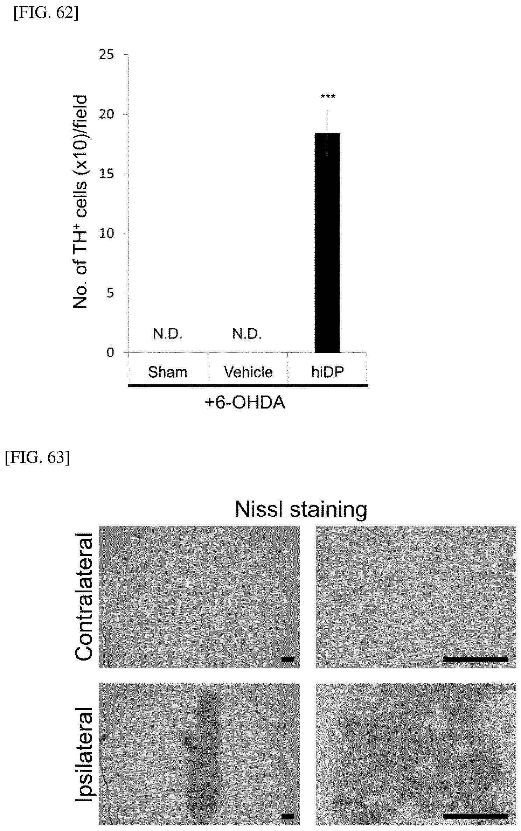

In order to directly evaluate the effect of hiDP transplantation, mice were euthanized for immunocytochemical staining for TH and DAT of striatum. No positive signal was found by the administration of 6-OHDA in the mock and vehicle control groups, but TH⁺DAT⁺ cells were found in the striatum of hiDPs-transplanted mice (FIGS. 61 and 62).

From these results, it was confirmed that the hiDPs of the present disclosure transplanted *in vivo* differentiate into functional DNs, which have a high possibility of contributing to recovery of exercise in a PD mouse model.

### Experimental Example 4.4. Evaluation of tumorigenicity of hiDP transplantation

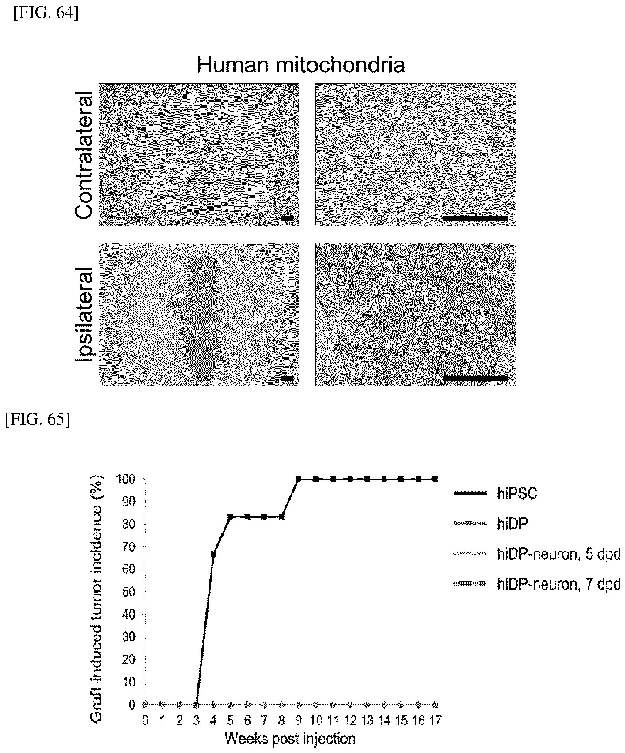

In order to further confirm the absence of tumorigenicity in the hiDPs-transplanted rats, hiDPs were transplanted into the striatum of normal rats while performing daily injection of an immunosuppressant. Through the results of Nissl staining for rat striatum and DBA staining for human-specific mitochondria, it was confirmed that the graft was maintained at the injection site without diffusion, the brain structure around the graft was not collapsed compared to the contralateral orientation, and included a polymorphism in terms of variability of cell size and shape, thus not showing neural rosettes or malignant tumors in the graft (FIGS. 63 and 64).

From these results, it was confirmed that tumors were not formed at the transplanted site when the hiDPs of the present disclosure were transplanted in normal rats, in consistent with the data in the PD mouse model.

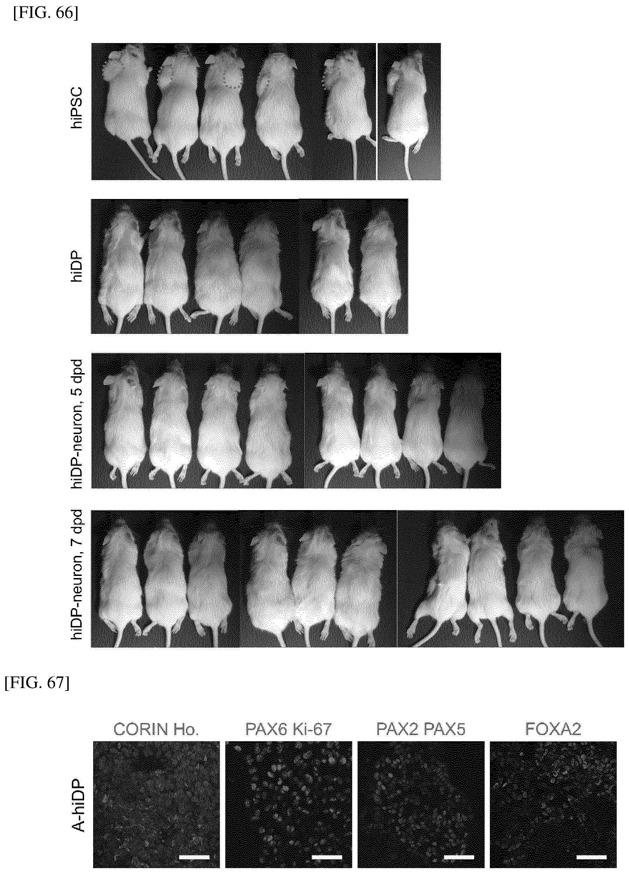

Additionally, the tumorigenicity of hiDPs was confirmed using immunodeficient NSG mice. Specifically, the hiDPs obtained in Example 1.2, differentiated hiDPs used for transplantation, and homogenous hiPSCs were injected subcutaneously into NSG mice. In the hiPSCs-injected group, tumors began to form at 4 weeks post-injection, and tumors were formed in all mice at the 9th week after the injection (FIGS. 65 and 66). In contrast, no tumors were formed in the hiDPs-injected group, regardless of the differentiation of hiDPs (FIGS. 65 and 66).

From these results, it was confirmed that the hiDP transplantation of the present disclosure has therapeutic efficacy against PD and safety against tumor formation.

### Experimental Example 5. Characterization of A-hiDPs produced from adult human fibroblasts

In order to confirm the expression of CORIN, PAX2, PAX5 and FOXA2 (which are representative DPs markers), Ki-67 (a cell cycle marker), and PAX6 (a key marker of hiNSCs) in the A-hiDPs obtained in Example 1.3, immunocytochemical staining was performed using the method described in Example 3. As a result, it was confirmed that CORIN, PAX2, PAX5, FOXA2, and Ki-67 were expressed, but PAX6 was not expressed (FIG. 67).

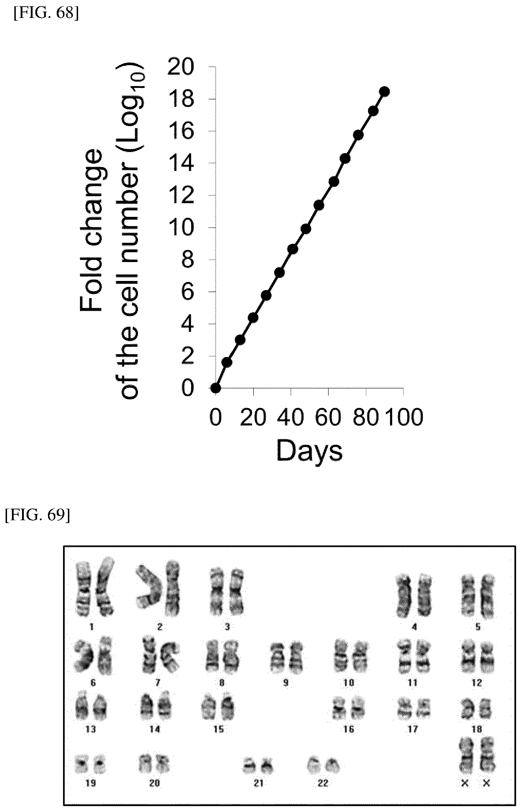

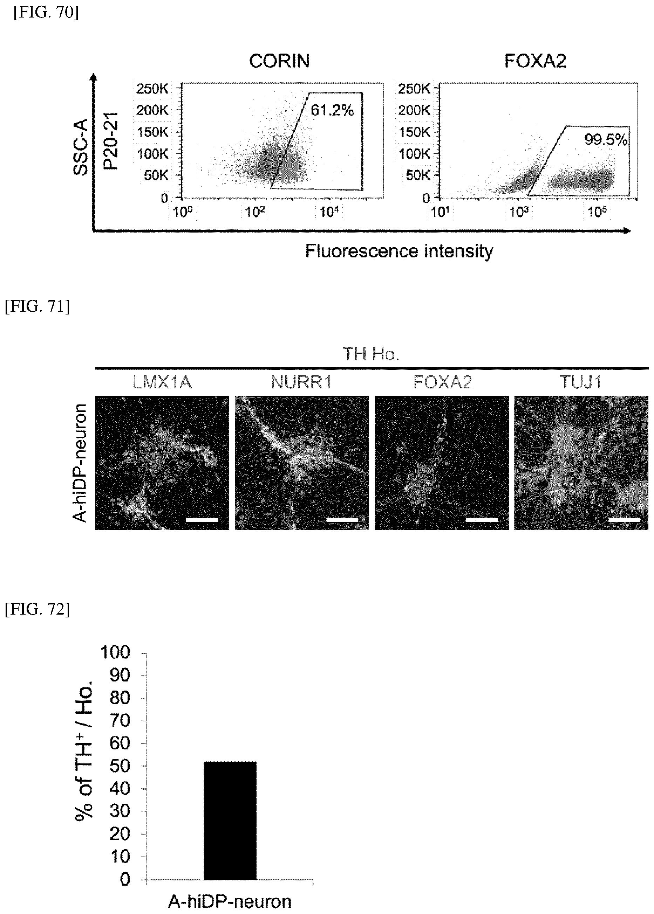

As a result of measuring the number of cells increased in the process of culturing A-hiDPs for 100 days or more, it was confirmed that the proliferation rate remained constant and the number of cells proliferated 10²⁰-fold or higher, thus confirming the possibility of proliferation (FIG. 68). In addition, through the karyotype analysis results, it was confirmed that there was no structural abnormality in the chromosome of A-hiDPs even after 20 or more times of subcultures (FIG. 69), and it was confirmed through flow cytometry that the expression of CORIN and FOXA2, which are key markers of DPs, was maintained at a high level (61.2% and 99.5%, respectively) even after 20 or more times of subcultures (FIG. 70).

In order to confirm the ability to differentiate into DNs, A-hiDPs were cultured under the neuronal differentiation conditions described in Experimental Example 3.1. TH⁺ neurons differentiated from A-hiDP were co-stained with LMX1A, NURR1, and FOXA2, which showed midbrain-specific features (FIG. 71). In addition, A-hiDPs produced TH⁺ neurons in high yield (52%) (FIG. 72).

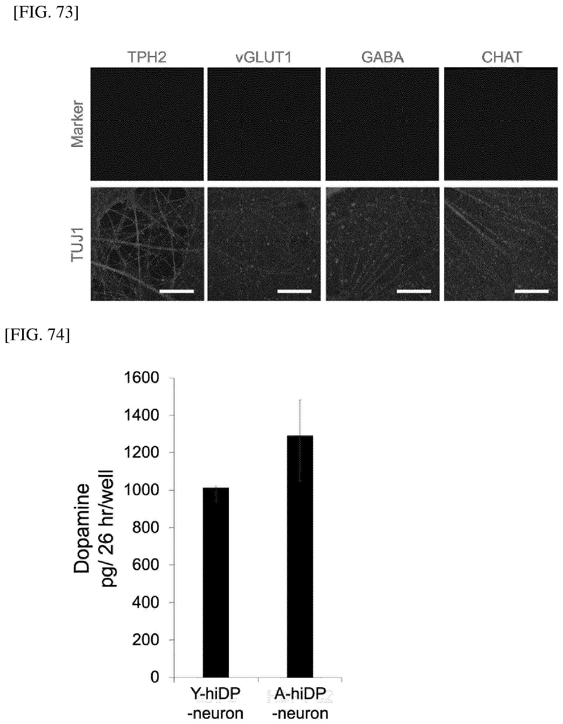

To investigate the proportion of different neuronal subtypes of the DNs differentiated from A-hiDPs, immunocytochemical staining was performed for serotonergic (TPH2), glutamatergic (vGLUT1), GABAergic (GABA), and cholinergic (CHAT) neuronal specific markers. As a result, it was confirmed that the corresponding cells were not present (FIG. 73).

The DNs differentiated from A-hiDPs were compared with the DNs differentiated from the hiDPs (Y-hiDPs) obtained in Example 1.2, and it was confirmed that similar amounts of dopamine were secreted (FIG. 74).

From these results, the reproducibility of the direct reprogramming protocol for hiDPs described in Example 1.2 was confirmed.

### Experimental Example 6. Characterization of hiDPs produced from human peripheral blood mononuclear cells

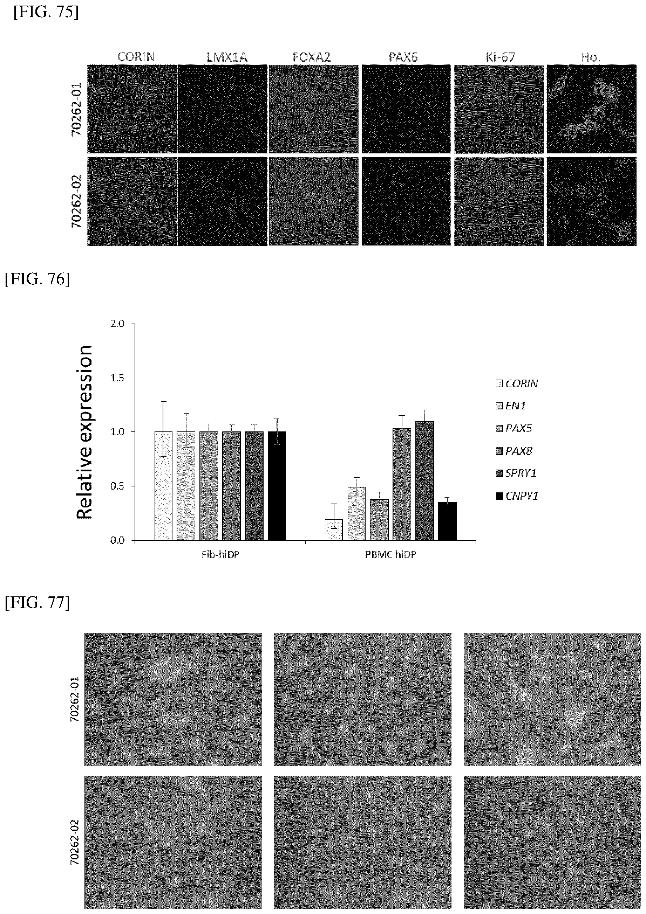

In order to confirm the expression of CORIN, LMX1A, and FOXA2 (which are representative DPs markers), Ki-67 (a cell cycle marker), and PAX6 (a key marker of hiNSCs) in the PBMC-hiDPs (70262-01 and 70262-02) obtained in Example 1.4, immunocytochemical staining was performed using the method described in Example 3. As a result, it was confirmed that CORIN, FOXA2, and Ki-67 were expressed, but LMX1A and PAX6 were not expressed (FIG. 75).

In addition, in order to compare the expression levels of midbrain DPs-specific markers (CORIN, EN1, PAX5, PAX8, SPRY1, and CNPY1) in PBMC-hiDPs with those of the fibroblast-derived hiDPs (Fb-hiDPs) obtained in Example 1.2, QRT-PCR was performed using the method described in Example 2. As a result, it was confirmed that there was no significant difference in the expression levels of CORIN, EN1, PAX5, PAX8, SPRY1, and CNPY1 compared to Fb-hiDPs (FIG. 76).

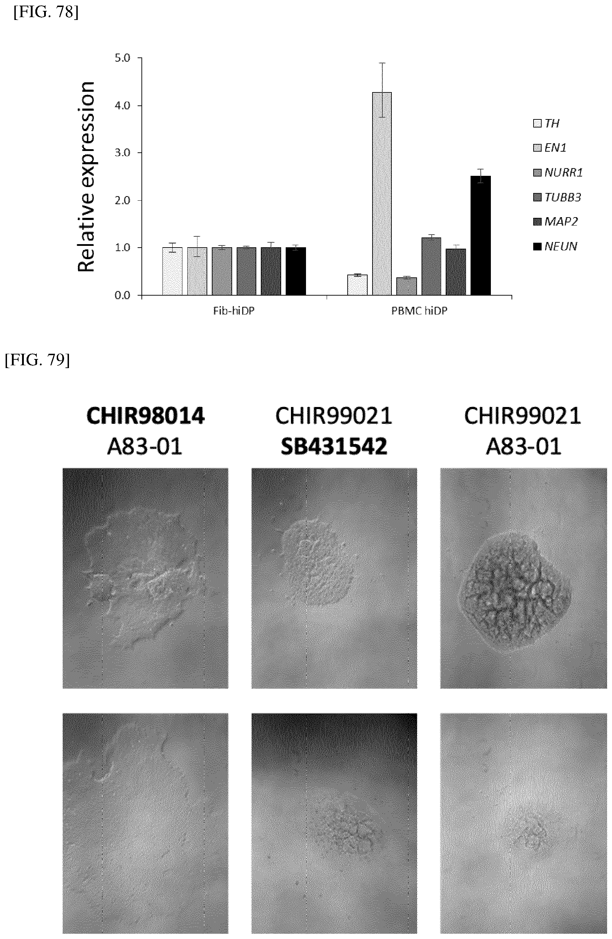

In order to confirm the ability to differentiate into DNs, A-hiDPs were cultured under the neuronal differentiation conditions described in Experimental Example 3.1. As a result of taking a bright field image on day 21 after differentiation, it was confirmed that the A-hiDPs were differentiated into a neuron-specific form (FIG. 77). In addition, qRT-PCR was performed to measure the expression levels of midbrain DNs-specific markers (TH, EN1, and NURR1) and markers associated with neuronal maturation (MAP2, NeuroN; NEUN), compared to Fb-hiDPs, and as a result, it was confirmed that there was no significant difference (FIG. 78).

From these results, it was confirmed that the direct reprogramming protocol of hiDPs described in Example 1.2 can be applied equally to other somatic cells including PBMCs as well as to fibroblasts.

### Experimental Example 7. Direct reprogramming of hiDPs using a low molecular weight compound replacing CHIR99021 and A83-01

Direct reprogramming of hiDPs was performed using the same direct reprogramming protocol for hiDPs described in Example 1.2, but using other small molecule compounds which replaced CHIR99021 (a WNT signaling agonist) or A83-01 (a TGF-β inhibitor). Specifically, 3.0 µM CHIR99021 (Stemcell Technologies) was replaced with 2.0 µM CHIR98014, or 0.5 µM A83-01 was replaced with 2.0 µM SB431542.

As a result of performing direct reprogramming protocol of hiDPs using the protocol described in Example 1.2 and each modified protocol, it was confirmed that cells in the form of hiDPs were produced under all conditions (FIG. 79).

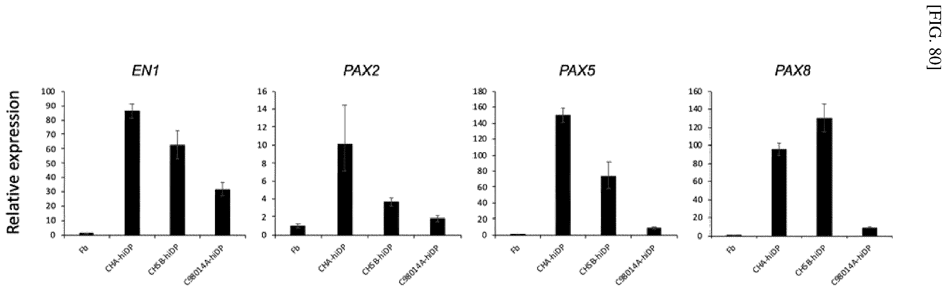

In order to compare the expression levels of midbrain DPs-specific markers (EN1, PAX2, PAX5, and PAX8) in parental fibroblasts and the hiDPs formed under each condition, qRT-PCR was performed using the method described in Example 2. As a result, it was confirmed that the expression level of all four genes was increased compared to that of the parental fibroblasts (FIG. 80).

From these results, it was confirmed that in performing the direct reprogramming protocol for hiDPs described in Example 1.2, direct reprogramming of hiDPs can be successfully performed even when other low-differentiating compounds, which are known to exhibit the effects of WNT signaling agonists and TGF-β inhibitors were used.

### Experimental Example 8. Bypassing pluripotent step during process of reprogramming hiDPs

Since reprogramming of hiDPs depends on ectopic expression of OSKM, which is a pluripotent inducer, there is a possibility that the hiDPs obtained through Example 1 were produced through a differentiation process after transient pluripotency acquisition. To test this possibility, qRT-PCR was performed using the method described in Example 2 and the expression levels of key marker genes of hiDPs, hiNSCs, and hiPSCs were compared, respectively.

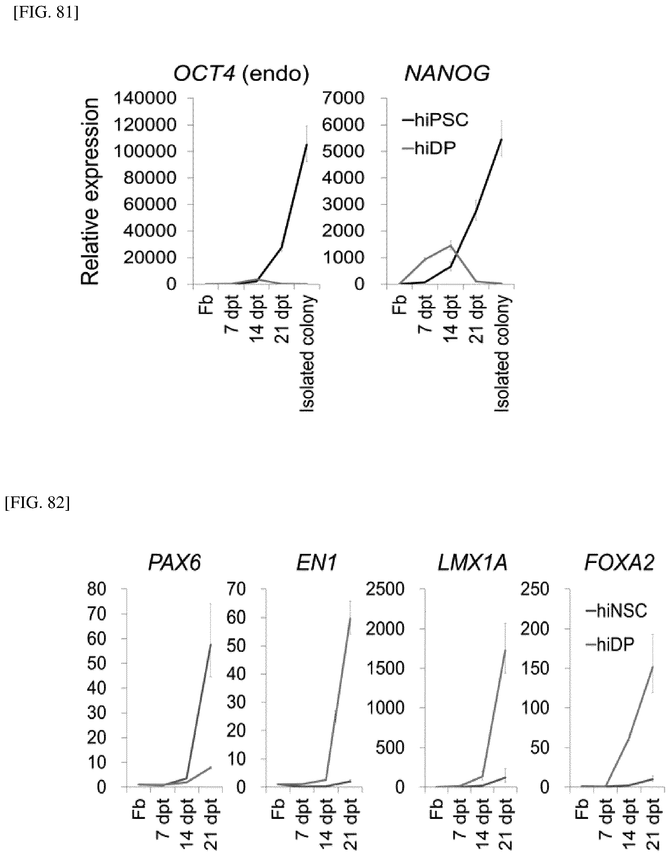

As a result of analyzing the key markers of hiPSCs, it was confirmed that the expression level of endogenous OCT4 was upregulated from 14 dpt of hiPSC reprogramming, but it did not change throughout the reprogramming process of hiDPs; and the expression level of NANOG was upregulated throughout the entire process of hiPSC reprogramming, but was temporarily increased at 7-14 dpt of hiDP reprogramming and then decreased again, which was at a low level compared to that of the finally obtained hiPSCs (FIG. 81).

The expression of PAX6, one of the key markers of hiNSCs, was first detected at 14 dpt of hiNSC reprogramming, and then continued to increase during hiNSC reprogramming (FIG. 82). As a result of analyzing the key markers of hiDPs, it was confirmed that the expression levels of EN1, LMX1A, and FOXA2 increased at 14 dpt or earlier of hiDP reprogramming, but the expression level was very low throughout the hiNSC reprogramming process (FIG. 82). From these results, it was confirmed that the hiDP reprogramming process is separate from the reprogramming pathways for hiPSCs and hiNSCs.

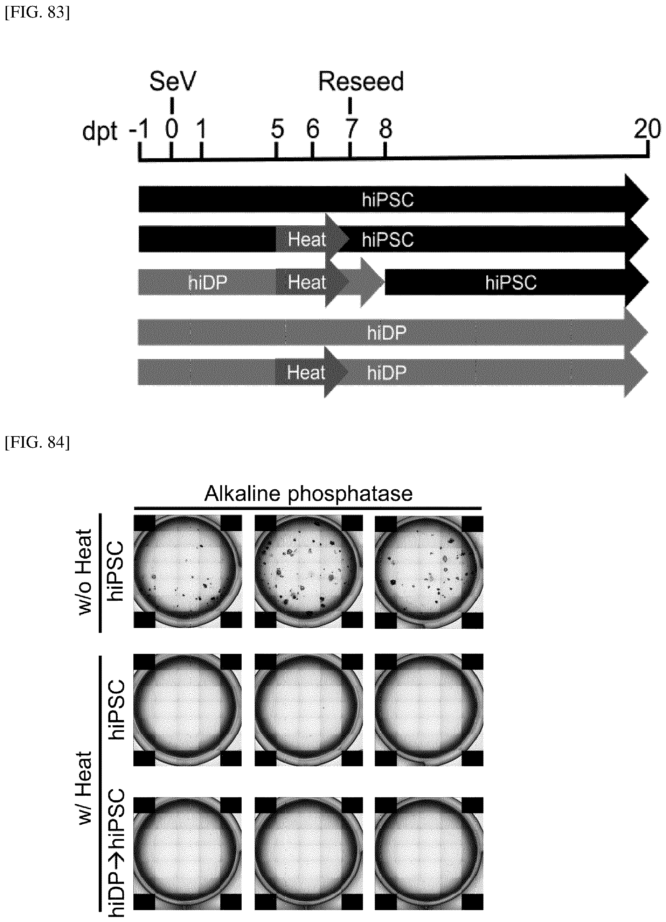

Meanwhile, since the hiDP reprogramming uses OSKM, there is a possibility that pluripotent cells are temporarily produced during the reprogramming process and re-differentiated later to produce hiDPs. In order to test this possibility, considering that SeV used in Example 1 and Comparative Examples 1 and 2 has a property of being inactivated by heat, the expression of OSKM was reduced to a level at which hiPSCs could not be produced by heat shock during the reprogramming process, and then, reprogramming was performed for the hiDPs of Example 1 and the hiPSC of Comparative Example 2 (FIG. 83).

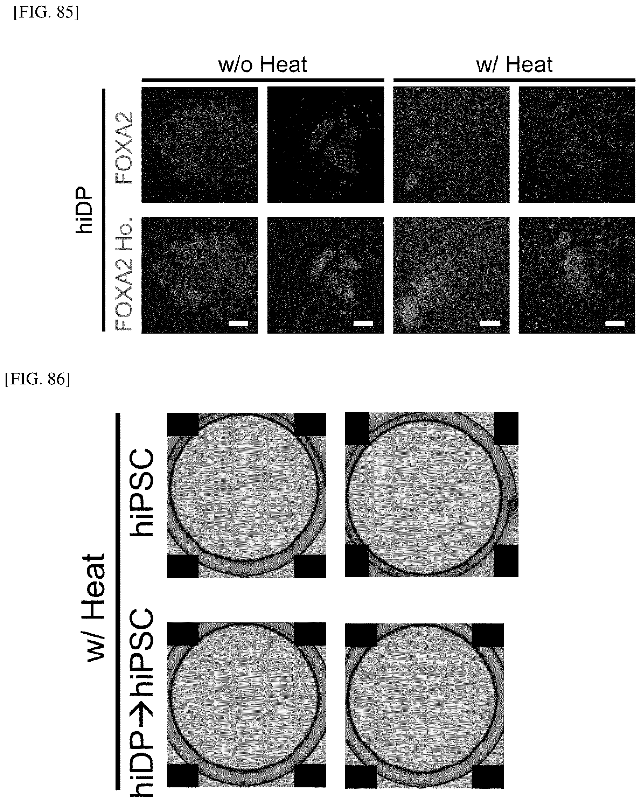

While ALP⁺ colonies were not observed in hiPSCs on day 20 under heat shock of 5 dpt to 7 dpt, ALP⁺ colonies were produced from the control cells without treatment of heat shock (FIG. 84). In addition, as a result of reprogramming of hiDPs under heat shock conditions in which ALP⁺ cell production was impossible, FOXA2⁺ colonies were detected (FIG. 85), which indicated that the cell fate of these colonies was converted to hiDPs.

Additionally, when hiDPs were produced from pluripotent intermediates, it was assumed that the intermediates undergoing reprogramming of hiDPs could be converted to hiPSCs along with the changes into a culture environment that induces pluripotency.

When heat shock was applied for 48 hours in the process of reprogramming hiDPs, even if the culture conditions for reprogramming hiDPs were changed to the culture conditions for reprogramming hiPSCs at the time point of 8 dpt, ALP⁺ colonies were not detected (FIG. 84). Since there is a possibility that the reprogramming process could have been delayed due to cell aging by heat shock, the reprogramming conditions were maintained from the existing 20 days to 30 days, but ALP⁺ colonies were still not detected (FIG. 86).

From these results, it was confirmed that the reprogramming of the hiDPs of Example 1 enables direct production from somatic cells without undergoing the pluripotent intermediate step.

### Experimental Example 9. Application of direct reprogramming for mouse iDPs to human fibroblasts

The present inventors have examined whether the conditions previously used for directly reprogramming of mouse fibroblasts into iDPs could be used in human fibroblasts so as to produce human induced dopaminergic neuron precursors (hiDPs).

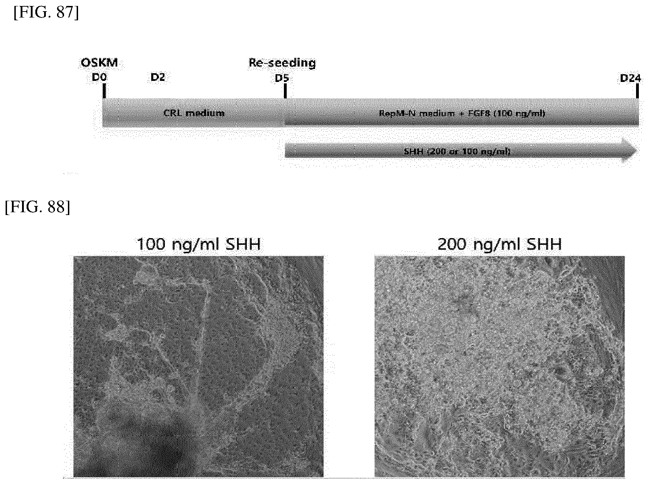

Human fibroblasts (CRL2097) were inoculated into a culture vessel coated with Matrigel (BD Biosciences, USA), and cultured in DMEM (Dulbecco's modified Eagle's medium) containing 10% FBS, 1% non-essential amino acids (NEAA), and 1% penicillin/streptomycin (P/S), and then the OSKM factor was transduced (D0). On day 6 (D5) after the transduction, cells were cultured in the RepM-N medium, which contained 200 ng/mL SHH or 100 ng/mL SHH and 100 ng/mL FGF8 as an inducing factor in a mixed medium where Advanced DMEM/F12 (which contained IX N2, IX B27, 0.05% BSA, 2 mM GlutaMax, and 0.11 mM beta-mercaptoethanol) and a neurobasal medium were mixed at a 1: 1 ratio, and cultured further for 19 days (FIG. 87).

As a result, it was confirmed that under both conditions using 200 ng/mL and 100 ng/mL of SHH, only cells in a form that cannot be seen as cells of the neuronal lineage including hiDPs were produced (FIG. 88).

From these results, it was confirmed that the direct reprogramming conditions of mouse iDPs have no effect on direct reprogramming of human fibroblasts into hiDPs.

## Claims

1. A method for preparing induced dopaminergic neuronal progenitors (iDPs), comprising:
a) introducing one or more genes selected from the group consisting of Oct4, Sox2, Klf4, and Myc into adult cells;
b) culturing the cells in a medium comprising EGF and FGF2; and
c) culturing the cells in a medium comprising FGF8, SHH, a Wnt signaling agonist, and a TGF-β inhibitor.

2. The method of claim 1,
wherein the adult cells are fibroblasts, peripheral blood mononuclear cells (PBMC), or mesenchymal stem cells (MSCs).

3. The method of claim 1,
wherein the adult cells are derived from humans.

4. The method of claim 1,
wherein the Wnt signaling agonist is any one selected from the group consisting of:
i) Wnt1, Wnt2, Wnt2b, Wnt3, Wnt3a, Wnt4, Wnt5a, Wnt5b, Wnt6, Wnt7a, Wnt7b, Wnt8a, Wnt8b, Wnt9a, Wnt9b, Wnt10a, Wnt10b, Wnt11, and Wnt16b;
ii) GSK3 inhibitors, which are lithium, LiCl, divalent zinc, BIO, SB216763, SB415286, CHIR99021, CHIR98014, a QS11 hydrate, TWS119, kenpaullone, alsterpolon, indirubin-3'-oxime, TDZD-8, and Ro 31-8220 methanesulfonate, which are GSK3 inhibitors;
iii) an Axin inhibitor;
iv) an APC inhibitor;
v) Norrin;
vi) R-spondin 2; and
a combination thereof.

5. The method of claim 1,
wherein the TGF-β inhibitor is any one selected from the group consisting of A83-01, SB431542, RepSox, LY364947, SB525334, and a combination thereof.

6. The method of claim 1,
wherein the method further comprises culturing by adding Y-27632, which is a rho-associated protein kinase (ROCK) inhibitor, after the culturing in Step b).

7. The method of claim 1,
wherein the medium in Step b) further comprises one or more compounds selected from the group consisting of a Wnt signaling agonist, a TGF-β inhibitor, 2-phospho-L-ascorbic acid, and sodium butyrate (NaB).

8. The method of claim 1,
wherein the medium in Step b) further comprises a Wnt signaling agonist and a TGF-β inhibitor.

9. The method of claim 1,
wherein the medium in Step b) further comprises 2-phospho-L-ascorbic acid.

10. The method of claim 1,
wherein the medium in Step b) comprises 1 ng/mL to 100 ng/mL of EGF and 1 ng/mL to 100 ng/mL of FGF2.

11. The method of claim 1,
wherein the medium in Step c) comprises 10 ng/mL to 1,000 ng/mL of FGF8, 100 ng/mL to 2,000 ng/mL of SHH, 0.1 µM to 50.0 µM of a Wnt signaling agonist, and 0.01 µM to 10.0 µM of a TGF-β inhibitor.

12. The method of claim 1,
wherein Step a) is performed for 12 hours to 36 hours.

13. The method of claim 1,
wherein Step b) is performed for 5 to 9 days.

14. The method of claim 1,
wherein Step c) is performed for 10 to 18 days.

15. The method of claim 1,
wherein the induced dopaminergic neuronal progenitors undergo direct reprogramming from adult cells.

16. An induced dopaminergic neuronal progenitor, which is prepared by the method of claim 1.

17. The induced dopaminergic neuronal progenitor of claim 16,
Which is proliferable.

18. An induced dopaminergic neuronal progenitor, wherein:
(i) one or more genes selected from the group consisting of CORIN, FOXA2, and LMX1A exhibit reduced expression compared to dopaminergic neuronal progenitors derived from pluripotent stem cells;
(ii) one or more genes selected from the group consisting of EN1, PAX2, PAX5, PAX8, and SPRY1 exhibit increased expression compared to dopaminergic neuronal progenitors derived from pluripotent stem cells; or
(iii) the reduced expression of (i) and the increased expression of (ii) are exhibited.

19. The induced dopaminergic neuronal progenitor of claim 18,
wherein the (i) one or more genes selected from the group consisting of CORIN, FOXA2, and LMX1A exhibit 1,000- to 100,000-fold reduced expression compared to dopaminergic neuronal progenitors derived from pluripotent stem cells.

20. The induced dopaminergic neuronal progenitor of claim 18,
wherein the (ii) one or more genes selected from the group consisting of EN1, PAX2, PAX5, PAX8, and SPRY1 exhibit 3- to 300-fold increased expression compared to dopaminergic neuronal progenitors derived from pluripotent stem cells.

21. The induced dopaminergic neuronal progenitor of claim 18,
wherein HOXB1 is not exhibited.

22. The induced dopaminergic neuronal progenitor of claim 18,
wherein endogenous Oct4 and NANOG exhibit reduced expression compared to induced pluripotent stem cells.

23. The induced dopaminergic neuronal progenitor of claim 18,
wherein PAX6 exhibits reduced expression compared to induced neural stem cells, and
EN1, LMX1A, and FOXA2 exhibit increased expression compared to induced neural stem cells.

24. A pharmaceutical composition for preventing or treating Parkinson's disease comprising the induced dopaminergic neuronal progenitors according to any one of claims 16 to 23 as an active ingredient.

25. A method for preventing or treating Parkinson's disease comprising administering to a subject in a therapeutically effective amount the pharmaceutical composition according to claim 24.

26. Use of the induced dopaminergic neuronal progenitors according to any one of claims 16 to 23 for preventing or treating Parkinson's disease.

27. Use of the induced dopaminergic neuronal progenitors according to any one of claims 16 to 23 for the preparation of a medicament for preventing or treating Parkinson's disease.

28. A method for screening agents for preventing or treating Parkinson's disease, comprising:
treating the induced dopaminergic neuronal progenitors according to any one of claims 16 to 23 with a candidate material for preventing or treating Parkinson's disease; and
measuring the proliferation ability, activity, or differentiation ability into dopaminergic neurons of the induced dopaminergic neuronal progenitors, compared to the control group not treated with the candidate material.

29. A mixture for preparing an induced dopaminergic neuronal progenitor, which comprises human adult cells into which one or more genes selected from the group consisting of Oct4, Sox2, Klf4, and Myc are introduced; EGF; FGF2; a Wnt signaling agonist; and a TGF-β inhibitor.

30. A mixture comprising an induced dopaminergic neuronal progenitor, FGF8, SHH, a Wnt signaling agonist, and a TGF-β inhibitor.

31. A medium composition for preparing an induced dopaminergic progenitor, which comprises EGF, FGF2, a Wnt signaling agonist, and a TGF-β inhibitor.

32. A medium composition for preparing or maintaining an induced dopaminergic progenitor, which comprises FGF8, SHH, a Wnt signaling agonist, and a TGF-β inhibitor.

33. A method for preparing a dopaminergic neuron, which comprises culturing the induced dopaminergic neuronal progenitor according to any one of claims 16 to 23 in a neuronal differentiation medium.


<110>    KOREA RESEARCH INSTITUTE OF BIOSCIENCE AND BIOTECHNOLOGY

<120>    METHOD FOR PRODUCING INDUCED DOPAMINEGERIC NEURONAL PROGENITORS
         BY DIRECT REPROGRAMMING

<130>    PCC007053KBB

<150>    KR 10-2019-0116258
<151>    2019-09-20

<160>    30

<170>    KoPatentIn 3.0

<210>    1
<211>    21
<212>    DNA
<213>    Artificial Sequence

<220>
<223>    FOXA2_F


<400>    1
ggagcagcta ctatgcagag c                                                  21


<210>    2
<211>    20
<212>    DNA
<213>    Artificial Sequence

<220>
<223>    FOXA2_R


<400>    2
cgtgttcatg ccgttcatcc                                                    20


<210>    3
<211>    19
<212>    DNA
<213>    Artificial Sequence

<220>
<223>    SHH_F


<400>    3
ctcgctgctg gtatgctcg                                                     19


<210>    4
<211>    21
<212>    DNA
<213>    Artificial Sequence

<220>
<223>    SHH_R


<400>    4

atcgctcgga gtttctggag a                                                21

<210>    5
<211>    21
<212>    DNA
<213>    Artificial Sequence

<220>
<223>    LMX1A_F

<400>    5
acgtccgaga accatcttga c                                                21

<210>    6
<211>    19
<212>    DNA
<213>    Artificial Sequence

<220>
<223>    LMX1A_R

<400>    6
caccaccgtt tgtctgagc                                                   19

<210>    7
<211>    19
<212>    DNA
<213>    Artificial Sequence

<220>
<223>    CORIN_F

<400>    7
ccaaagccgg tcttgagag                                                   19

<210>    8
<211>    19
<212>    DNA
<213>    Artificial Sequence

<220>
<223>    CORIN_R

<400>    8
gaggaggtta gcagtcgcc                                                   19

<210>    9
<211>    19
<212>    DNA
<213>    Artificial Sequence

<220>
<223>    EN1_F

<400>    9
cgcagcagcc tctcgtatg                                                    19


<210>    10
<211>    19
<212>    DNA
<213>    Artificial Sequence

<220>
<223>    EN1_R


<400>    10
cctggaactc cgccttgag                                                    19


<210>    11
<211>    22
<212>    DNA
<213>    Artificial Sequence

<220>
<223>    PAX2_F


<400>    11
acgaggcttg gagattcagc aa                                                22


<210>    12
<211>    21
<212>    DNA
<213>    Artificial Sequence

<220>
<223>    PAX2_R


<400>    12
tgtcggcctg aagcttgatg t                                                 21


<210>    13
<211>    21
<212>    DNA
<213>    Artificial Sequence

<220>
<223>    PAX5_F


<400>    13
acttgctcat caaggtgtca g                                                 21


<210>    14
<211>    21
<212>    DNA
<213>    Artificial Sequence

<220>

<223>    PAX5_R

<400>    14
tcctccaatt accccaggct t                                                21

<210>    15
<211>    22
<212>    DNA
<213>    Artificial Sequence

<220>
<223>    PAX8_F

<400>    15
atagctgccg actaagcatt ga                                               22

<210>    16
<211>    19
<212>    DNA
<213>    Artificial Sequence

<220>
<223>    PAX8_R

<400>    16
atccgtgcga aggtgcttt                                                   19

<210>    17
<211>    22
<212>    DNA
<213>    Artificial Sequence

<220>
<223>    SPRY1_F

<400>    17
gccctggata aggaacagct ac                                               22

<210>    18
<211>    22
<212>    DNA
<213>    Artificial Sequence

<220>
<223>    SPRY1_R

<400>    18
gccgaaatgc ctaatgcaaa ga                                               22

<210>    19
<211>    20
<212>    DNA
<213>    Artificial Sequence

<220>
<223>    Endo-OCT4_F

<400>    19
agtttgtgcc agggtttttg                                                    20

<210>    20
<211>    20
<212>    DNA
<213>    Artificial Sequence

<220>
<223>    Endo-OCT4_R

<400>    20
acttcacctt ccctccaacc                                                    20

<210>    21
<211>    20
<212>    DNA
<213>    Artificial Sequence

<220>
<223>    NANOG_F

<400>    21
aacgttctgc tggactgagc                                                    20

<210>    22
<211>    20
<212>    DNA
<213>    Artificial Sequence

<220>
<223>    NANOG_R

<400>    22
atgcttcaaa gcaaggcaag                                                    20

<210>    23
<211>    20
<212>    DNA
<213>    Artificial Sequence

<220>
<223>    PAX6_F

<400>    23
gtccatcttt gcttgggaaa                                                    20

<210>    24
<211>    20

<212>    DNA
<213>    Artificial Sequence

<220>
<223>    PAX6_R


<400>    24
tagccaggtt gcgaagaact                                                    20


<210>    25
<211>    21
<212>    DNA
<213>    Artificial Sequence

<220>
<223>    NURR1_F


<400>    25
accactcttc gggagaatac a                                                  21


<210>    26
<211>    21
<212>    DNA
<213>    Artificial Sequence

<220>
<223>    NURR1_R


<400>    26
ggcatttggt acaagcaagg t                                                  21


<210>    27
<211>    21
<212>    DNA
<213>    Artificial Sequence

<220>
<223>    HOXA2_F


<400>    27
cccctgtcgc tgatacattt c                                                  21


<210>    28
<211>    21
<212>    DNA
<213>    Artificial Sequence

<220>
<223>    HOXA2_R


<400>    28
tggtctgctc aaaaggagga g                                                  21

<210>    29
<211>    20
<212>    DNA
<213>    Artificial Sequence

<220>
<223>    GAPDH_F

<400>    29
tgcaccacca actgcttagc                                                    20

<210>    30
<211>    21
<212>    DNA
<213>    Artificial Sequence

<220>
<223>    GAPDH_R

<400>    30
ggcatggact gtggtcatga g                                                  21